# EUROPEAN PATENT SPECIFICATION

(11) **EP 2 540 701 B1**
(45) Date of publication and mention of the grant of the patent: **22.10.2014**
(21) Application number: 11747583.0
(22) Date of filing: 25.02.2011
(51) Int. Cl.: C07C 249/08, C07C 251/40, C07C 253/00, C07C 255/19, C07B 61/00

(54) **PROCESS FOR PRODUCTION OF CYCLOPROPANECARBOXYLIC ACID ESTER COMPOUND**
VERFAHREN ZUR HERSTELLUNG EINER CYCLOPROPANCARBONSÄUREESTERVERBINDUNG
PROCÉDÉ POUR LA PRODUCTION DE COMPOSÉ ESTER D'ACIDE CYCLOPROPANECARBOXYLIQUE

(30) Priority: 24.08.2010 JP 2010186897; 15.04.2010 JP 2010093830; 30.03.2010 JP 2010077188; 26.02.2010 JP 2010041892
(43) Date of publication of application: 02.01.2013
(73) Proprietor: Sumitomo Chemical Co., Ltd, Chuo-ku, Tokyo 104-8260 (JP)
(72) Inventor: YOSHIKAWA, Kouji, Toyonaka-shi Osaka 560-0021 (JP); KIJI, Toshiyuki, Nishinomiya-shi Hyogo 663-8247 (JP); OHSHITA, Jun, Nishinomiya-shi Hyogo 663-8245 (JP); SOMYO, Toshio, Ibaraki-shi Osaka 567-0841 (JP); KASHIWABARA, Manabu, Fukuoka-shi Fukuoka 812-0054 (JP); MIYANAGA, Yoko, Ibaraki-shi Osaka 567-0841 (JP)
(74) Representative: Vossius & Partner
(86) International application number: PCT/JP2011/055010
(87) International publication number: WO 2011/105632

(56) References cited:
- EP-A1- 2 537 828
- WO-A2-2008/032585
- WO-A2-2010/087419
- JP-A- 60 054 350
- JP-A- 2008 094 832
- DATABASE CA [Online] CHEMICAL ABSTRACTS SERVICE, COLUMBUS, OHIO, US; MATSUI, MASANAO ET AL: "2,2-Dimethyl - 3 - (2 - cyano - 1 - propenyl)cyclopropanecarboxylates", XP002712488, retrieved from STN Database accession no. 1963:461696 & JP 38 002272 B (SUMITOMO CHEMICAL INDUSTRY CO., LTD.) 18 March 1963 (1963-03-18)
- STREITWIESER: 'Yuki Kagaku Kaisetsu 1, 4th edition' KABUSHIKI KAISHA HIROKAWA SHOTEN 1995, page 446
- MARUZEN CO., LTD. JIKKEN KAGAKU KOZA 20 YUKI GOSEI II -ALCOHOLAMINE-, 4TH EDITION 1996, pages 451 - 452

## Description

### Technical Field

The present invention relates to a production method of a cyclopropanecarboxylic acid ester compound.

### Background Art

Cyclopropanecarboxylic acid ester compounds such as hydroxyiminoalkenylcyclopropanecarboxylic acid esters, cyanoalkenylcyclopropane compounds and the like are compounds important as a synthetic intermediate for pyrethroid type domestic pest preventive agents, insecticides and the like.

As a production method of a hydroxyiminoalkenylcyclopropanecarboxylic acid ester, there is known, for example, a method in which methyl 2,2-dimethyl-3-(2-formyl-1-propenyl)cyclopropanecarboxylate and hydroxylamine hydrochloride are reacted by heating under reflux in ethanol and pyridine (see, e.g., JP-B No. 38-2272, examples).

JP-B No. 38-2272 describes also a method in which methyl 2,2-dimethyl-3-[3-(hydroxyimino)-2-methyl-1-propenyl]cyclopropanecarboxylate and acetic anhydride are mixed and the resultant mixture is heated to boil, as a production method of a cyanoalkenylcyclopropane compound.

### Summary of the Invention

The present inventors have intensively studied a method of producing a hydroxyiminoalkenylcyclopropanecarboxylic acid ester from a formylalkenylcyclopropanecarboxylic acid ester, leading to the present invention.

That is, the present invention is as described below.
<1> A method comprising
   an oximation step of reacting a formylalkenylcyclopropanecarboxylic acid ester represented by the formula (1) : 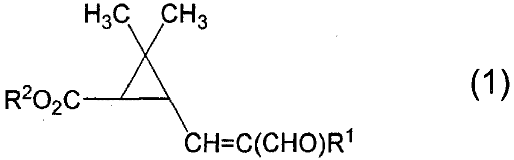
   (wherein, R¹ represents an alkyl group optionally having substituent(s) or halogen atom(s), and R² represents an alkyl group optionally having substituent(s) or a benzyl group optionally having substituent(s).)
   and hydroxylamine in a solvent in the presence at least one compound selected from the group consisting of carboxylic acids and carboxylic acid metal salts,
   to produce a hydroxyiminoalkenylcyclopropanecarboxylic acid ester represented by the formula (3): 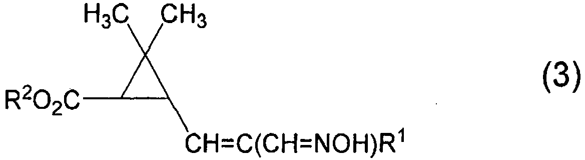 (wherein, R¹ and R² are as defined above.).
<2> The production method according to <1>, wherein the at least one compound selected from the group consisting of carboxylic acids and carboxylic acid metal salts is a carboxylic acid having 3 or more carbon atoms or a metal salt of a carboxylic acid having 3 or more carbon atoms.
<3> The production method according to <1> or <2>, wherein the solvent is a mixed solvent composed of water and an organic solvent immiscible with water.
<4> A method comprising
   an oximation step of reacting a formylalkenylcyclopropanecarboxylic acid ester represented by the formula (1) : 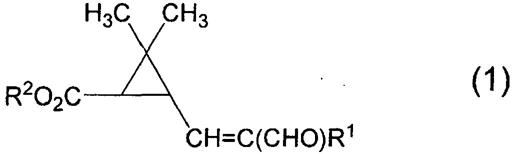
   (wherein, R¹ represents an alkyl group optionally having substituent(S) or halogen atom(s), and R² represents an alkyl group optionally having substituent(s) or a benzyl group optionally having substituent(s).)
   and hydroxylamine in a solvent in the presence of at least one compound selected from the group consisting of carboxylic acids and carboxylic acid metal salts,
   and a nitrilation step of reacting a hydroxyiminoalkenylcyclopropanecarboxylic acid ester represented by the formula (3): 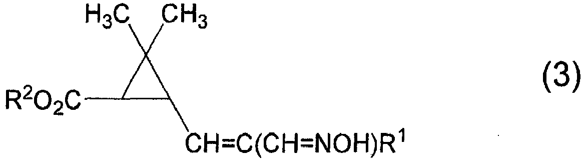 (wherein, R¹ and R² are as defined above.)
      obtained in the above-described oximation step and a dehydrating agent,
      to produce a cyanoalkenylcyclopropanecarboxylic acid ester represented by the formula (2): 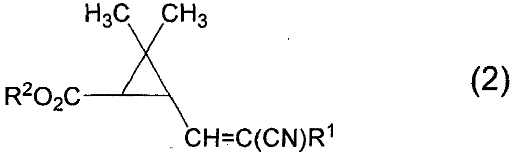 (wherein, R¹ and R² are as defined above.).
<5> The production method according to <4>, wherein the dehydrating agent is at least one compound selected from the group consisting of carboxylic anhydrides and acyl halides.
<6> The production method according to <4> or <5>, wherein the dehydrating agent is a carboxylic anhydride.
<7> The production method according to any one of <4> to <6>, wherein the nitrilation step is carried out in the presence of at least one acid selected from the group consisting of hydrogen halides and organic sulfonic acids.
<8> The production method according to any one of <4> to <7>, wherein the nitrilation step is carried out in the presence of an organic base.
<9> The production method according to any one of <4> to <8>, further comprising a step of removing at least one compound selected from the group consisting of carboxylic acids and carboxylic acid metal salts from a mixture obtained by the reaction in the nitrilation step.
<10> The production method according to <9>, wherein the at least one compound selected from the group consisting of carboxylic acids and carboxylic acid metal salts is removed into an alkali aqueous solution.
<11> The production method according to <10>, wherein the alkali aqueous solution is an alkali aqueous solution having pH of 10 or more.
<12> The production method according to <10> or <11>, wherein the alkali aqueous solution is an alkali metal hydroxide aqueous solution or an alkali metal carbonate aqueous solution.
<13> A method comprising
   a step of reacting a hydroxyiminoalkenylcyclopropane compound represented by the formula (3): 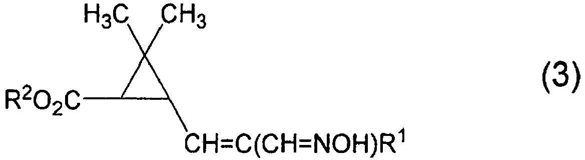 (wherein, R¹ represents an alkyl group optionally having substituent(s) or halogen atom(s), and R² represents an alkyl group optionally having substituent(s) or a benzyl group optionally having substituent(s).)
      and a carboxylic anhydride in the presence of an organic base and at least one acid selected from the group consisting of hydrogen halides and organic sulfonic acids,
      to produce a cyanoalkenylcyclopropane compound represented by the formula (2): 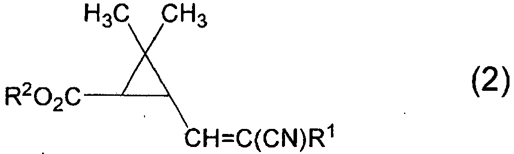 (wherein, R¹ and R² are as defined above.).
<14> The production method according to <13>, wherein the above-described step is a step of reacting a hydroxyiminoalkenylcyclopropane compound represented by the formula (1) and a carboxylic anhydride in the presence of a solvent.
<15> The production method according to <13> or <14>, wherein the organic base forms a salt of the above-described acid with the organic base.
<16> The production method according to any one of <13> to <15>, wherein the carboxylic anhydride is a carboxylic anhydride having 2 to 8 carbon atoms.
<17> The production method according to any one of <13> to <16>, wherein the carboxylic anhydride is acetic anhydride.
<18> The production method according to any one of <13> to <17>, wherein the organic base is at least one selected from the group consisting of pyridine, methylethylpyridine, dimethylaminopyridine and triethylamine.
<19> The production method according to any one of <13> to <18>, wherein the organic base is pyridine.
<20> The production method according to any one of <13> to <19>, wherein the above-described acid is a hydrogen halide.
<21> The production method according to any one of <13> to <20>, wherein the above-described acid is hydrogen chloride.
<22> The production method according to any one of <13> to <21>, wherein the above-described acid is an acid generated from at least one compound selected from the group consisting of carboxylic halides, organic sulfonic halides, metal halides and thionyl halides.

### Modes for Carrying Out the Invention

First, the formylalkenylcyclopropanecarboxylic acid ester represented by the formula (1) (hereinafter, referred to as formyl compound (1) in some cases.) will be explained.

For a geometric isomer based on a double bond, the formyl compound (1) may be and E form or a Z form, or may also be a mixture of an E form an a Z form at any ratio.

For a geometric isomer based on a cyclopropane ring, the formyl compound (1) may be a cis form or a trans form, or may also be a mixture of a cis form and a trans form at any ratio.

In the formyl compound (1), two carbon atoms among carbon atoms forming a cyclopropane ring are asymmetric carbon atoms, and the formyl compound (1) may be a compound showing optical activity or a compound showing no optical activity.

In the formyl compound (1), R¹ represents an alkyl group optionally having substituent(s) or halogen atom(s).

The halogen atom includes a fluorine atom, a chlorine atom, a bromine atom, an iodine atom and the like, and the halogen atom is preferably a chlorine atom or a bromine atom.

The alkyl group represented by R¹ includes linear alkyl groups having about 1 to 10 carbon atoms, branched alkyl groups having about 3 to 10 carbon atoms, cycloalkyl groups having about 3 to 10 carbon atoms and the like.

The linear alkyl group having about 1 to 10 carbon atoms includes a methyl group, an ethyl group, a n-propyl group and the like, the branched alkyl group having about 3 to 10 carbon atoms includes an isopropyl group, an isobutyl group, a tert-butyl group, an isoamyl group, a 2-ethylhexyl group and the like, and the cycloalkyl group having about 3 to 10 carbon atoms includes a cyclopropyl group, a cyclopentyl group, a cyclohexyl group and the like.

The substituent carried on the alkyl group represented by R¹ includes halogen atoms such as a fluorine atom, a chlorine atom, a bromine atom and the like; alkoxy groups having about 1 to 4 carbon atoms such as a methoxy group, an ethoxy group and the like; etc. The alkyl group having substituent(s) includes a fluoromethyl group, a chloromethyl group, a trifluoromethyl group, a trichloromethyl group and the like.

R¹ includes preferably linear alkyl groups having 1 to 4 carbon atoms, a chlorine atom, a bromine atom and the like, more preferably a methyl group, a chlorine atom and the like.

In the formyl compound (1), R² represents an alkyl group optionally having substituent(s) or a benzyl group optionally having substituent(s).

As the alkyl group represented by R², those listed for the above-described alkyl group represented by R¹ are mentioned.

The substituent carried on the alkyl group represented by R² includes halogen atoms such as a fluorine atom, a chlorine atom, a bromine atom and the like; alkenyl groups having about 3 to 4 carbon atoms such as an allyl group and the like; alkynyl groups having about 3 to 4 carbon atoms such as a propargyl group and the like; alkoxy groups having about 1 to 4 carbon atoms such as a methoxy group, an ethoxy group and the like; alkylthio groups having about 1 to 4 carbon atoms such as a methylthio group and the like; etc.

The substituent carried on the above-described benzyl group includes, for example, halogen atoms such as a fluorine atom, a chlorine atom, a bromine atom and the like; alkenyl groups having about 3 to 4 carbon atoms such as an allyl group and the like; alkynyl groups having about 3 to 4 carbon atoms such as a propargyl group and the like; alkoxy groups having about 1 to 4 carbon atoms such as a methoxy group, an ethoxy group and the like; alkylthio groups having about 1 to 4 carbon atoms such as a methylthio group and the like; linear alkyl groups having about 1 to 10 carbon atoms such as a methyl group, an ethyl group, a n-propyl group and the like; branched alkyl groups having about 3 to 10 carbon atoms such as an isopropyl group, an isobutyl group, a tert-butyl group, an isoamyl group, a 2-ethylhexyl group and the like; cycloalkyl groups having about 3 to 10 carbon atoms such as a cyclopropyl group, a cyclopentyl group, a cyclohexyl group and the like; alkoxymethyl groups having about 2 to 4 carbon atoms such as a methoxymethyl group, an ethoxymethyl group and the like; alkylthiomethyl groups having about 1 to 4 carbon atoms such as a methylthiomethyl group and the like; etc.

R² includes preferably linear alkyl groups having 1 to 4 carbon atoms, benzyl groups optionally having substituent(s), and the like, more preferably linear alkyl groups having 1 to 4 carbon atoms; a benzyl group; and benzyl groups substituted by group(s) selected from the group consisting of halogen atoms, alkenyl groups having 3 to 4 carbon atoms, linear alkyl groups having 1 to 4 carbon atoms, alkoxymethyl groups having 2 to 4 carbon atoms and alkylthiomethyl groups having 1 to 4 carbon atoms, further preferably a methyl group, an ethyl group, a benzyl group, a 4-methyl-2,3,5,6-tetrafluorobenzyl group, a 4-methoxymethyl-2,3,5,6-tetrafluorobenzyl group, a 4-allyl-2,3,5,6-tetrafluorobenzyl group, a 4-propargyl-2,3,5,6-tetrafluorobenzyl group, a 4-methylthiomethyl-2,3,5,6-tetrafluorobenzyl group and the like.

As the formyl compound (1), compounds described in Tables 1, 2 and 3 are specifically exemplified.

**Table 1**

| | | | | | | | | |
|---|---|---|---|---|---|---|---|---|
| 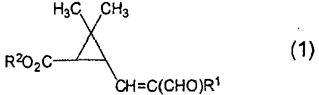 | | | | | | | | |

| Number | R¹ | R² | Number | R¹ | R² | Number | R¹ | R² |
|---|---|---|---|---|---|---|---|---|
| (1-1) | -CH₃ | -CH₃ | (1-21) | -CH₃ | -CH₂CH₃ | (1-41) | -CH₃ | 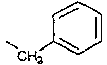 |
| (1-2) | -CH₂CH₃ | -CH₃ | (1-22) | -CH₂CH₃ | -CH₂CH₃ | (1-42) | -CH₂CH₃ | 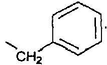 |
| (1-3) | -CH₂CH₂CH₃ | -CH₃ | (1-23) | -CH₂CH₂CH₃ | -CH₂CH₃ | (1-43) | -CH₂CH₂CH₃ | 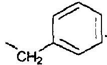 |
| (1-4) | 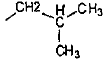 | -CH₃ | (1-24) | 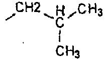 | -CH₂CH₃ | (1-44) | 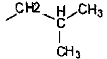 | 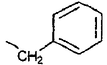 |
| (1-5) | 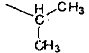 | -CH₃ | (1-25) | 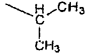 | -CH₂CH₃ | (1-45) | 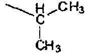 | 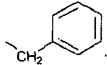 |
| (1-6) | 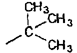 | -CH₃ | (1-26) | 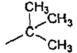 | -CH₂CH₃ | (1-46) | 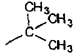 | 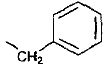 |
| (1-7) | -(CH₂)₄CH₃ | -CH₃ | (1-27) | -(CH₂)₄CH₃ | -CH₂CH₃ | (1-47) | -(CH₂)₄CH₃ | 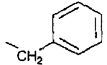 |
| (1-8) | 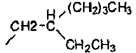 | -CH₃ | (1-28) | 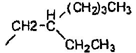 | -CH₂CH₃ | (1-48) | 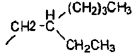 | 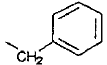 |
| (1-9) | 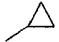 | -CH₃ | (1-29) | 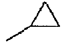 | -CH₂CH₃ | (1-49) | 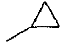 | 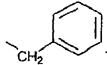 |
| (1-10) | 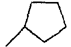 | -CH₃ | (1-30) | 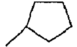 | -CH₂CH₃ | (1-50) | 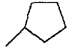 | 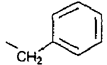 |
| (1-11) | 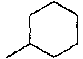 | -CH₃ | (1-31) | 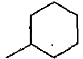 | -CH₂CH₃ | (1-51) | 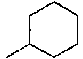 | 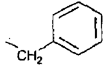 |
| (1-12) | -F | -CH₃ | (1-32) | -F | -CH₂CH₃ | (1-52) | -F | 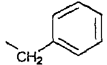 |
| (1-13) | -Cl | -CH₃ | (1-33) | -Cl | -CH₂CH₃ | (1-53) | -Cl | 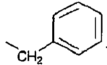 |
| (1-14) | -Br | -CH₃ | (1-34) | -Br | -CH₂CH₃ | (1-54) | -Br | 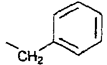 |
| (1-15) | -I | -CH₃ | (1-35) | -I | -CH₂CH₃ | (1-55) | -I | 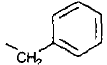 |

**Table 2**

| | | | | | | | | |
|---|---|---|---|---|---|---|---|---|
| 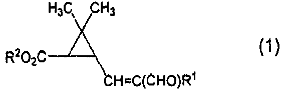 | | | | | | | | |

| Number | R¹ | R² | Number | R¹ | R² | Number | R¹ | R² |
|---|---|---|---|---|---|---|---|---|
| (1-61) | -CH₃ | 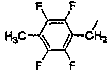 | (1-81) | -CH₃ | 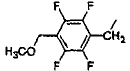 | (1-101) | -CH₃ | 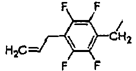 |
| (1-62) | -CH₂CH₃ | 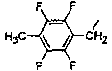 | (1-82) | -CH₂CH₃ | 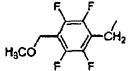 | (1-102) | -CH₂CH₃ | 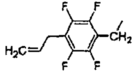 |
| (1-63) | -CH₂CH₂CH₃ | 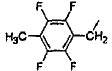 | (1-83) | -CH₂CH₂CH₃ | 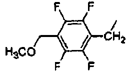 | (1-103) | -CH₂CH₂CH₃ | 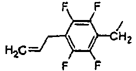 |
| (1-84) | 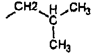 | 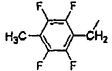 | (1-84) | 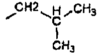 | 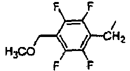 | (1-104) | 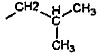 | 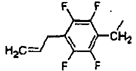 |
| (1-65) | 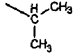 | 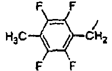 | (1-85) | 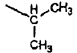 | 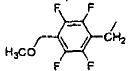 | (1-105) | 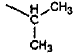 | 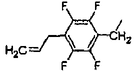 |
| (1-86) | 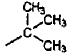 | 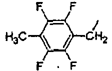 | (1-86) | 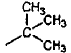 | 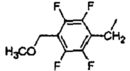 | (1-106) | 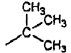 | 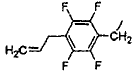 |
| (1-67) | -(CH₂)₄CH₃ | 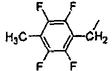 | (1-87) | -(CH₂)₄CH₃ | 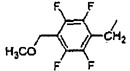 | (1-107) | -(CH₂)₄CH₃ | 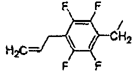 |
| (1-68) | 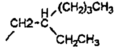 | 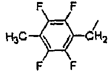 | (1-88) | 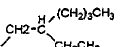 | 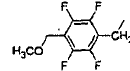 | (1-108) | 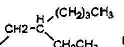 | 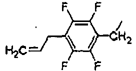 |
| (1-69) | 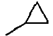 | 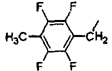 | (1-89) | 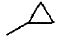 | 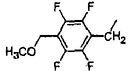 | (1-109) | 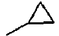 | 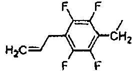 |
| (1-70) | 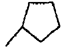 | 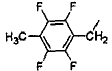 | (1-90) | 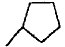 | 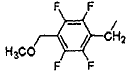 | (1-110) | 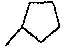 | 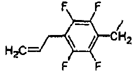 |
| (1-71) | 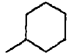 | 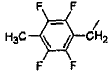 | (1-91) | 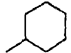 | 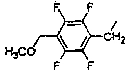 | (1-111) | 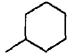 | 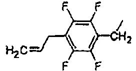 |
| (1-72) | -F | 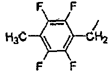 | (1-92) | -F | 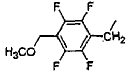 | (1-112) | -F | 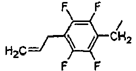 |
| (1-73) | -Cl | 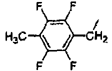 | (1-83) | -Cl | 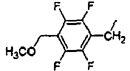 | (1-113) | -Cl | 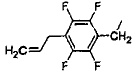 |
| (1-74) | -Br | 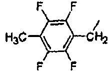 | (1-94) | -Br | 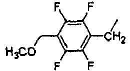 | (1-114) | -Br | 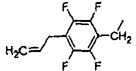 |
| (1-75) | -I | 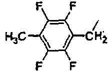 | (1-95) | -I | 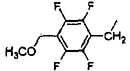 | (1-115) | -I | 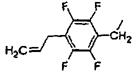 |

**Table 3**

| | | | | | |
|---|---|---|---|---|---|
| 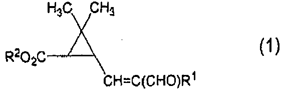 | | | | | |

| Number | R¹ | R² | Number | R¹ | R² |
|---|---|---|---|---|---|
| (1-116) | -CH₃ | 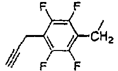 | (1-131) | -CH₃ | 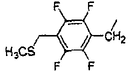 |
| (1-117) | -CH₂CH₃ | 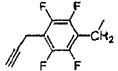 | (1-132) | -CH₂CH₃ | 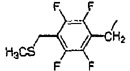 |
| (1-118) | -CH₂CH₂CH₃ | 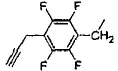 | (1-133) | -CH₂CH₂CH₃ | 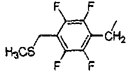 |
| (1-119) | 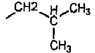 | 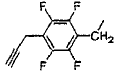 | (1-134) | 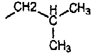 | 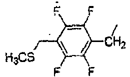 |
| (1-120) | 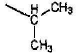 | 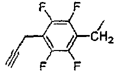 | (1-135) | 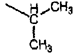 | 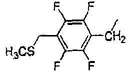 |
| (1-121) | 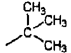 | 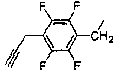 | (1-138) | 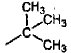 | 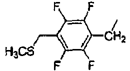 |
| (1-122) | -(CH₂)₄CH₃ | 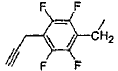 | (1-137) | -(CH₂)₄CH₃ | 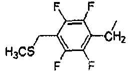 |
| (1-123) | 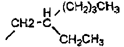 | 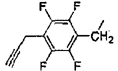 | (1-138) | 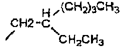 | 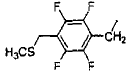 |
| (1-124) | 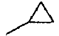 | 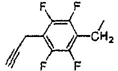 | (1-139) | 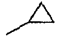 | 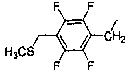 |
| (1-125) | 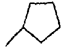 | 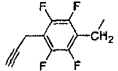 | (1-140) | 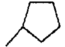 | 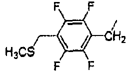 |
| (1-126) | 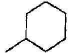 | 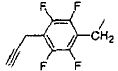 | (1-141) | 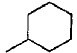 | 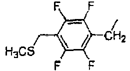 |
| (1-127) | -F | 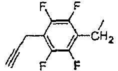 | (1-142) | -F | 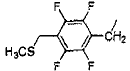 |
| (1-128) | -Cl | 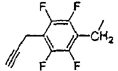 | (1-143) | -Cl | 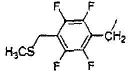 |
| (1-129) | -Br | 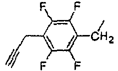 | (1-144) | -Br | 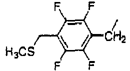 |
| (1-130) | -I | 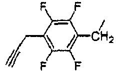 | (1-145) | -I | 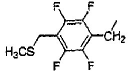 |

As the formyl compound (1), compounds represented by the number (1-1), (1-13), (1-21), (1-33), (1-61), (1-73), (1-81) or (1-93) in the tables are preferable, compounds represented by the number (1-1), (1-21), (1-61) or (1-81) in the tables are more preferable.

The formyl compound (1) can be produced, for example, by a method described in JP-A No. 2008-44900. Specifically, a method in which a compound represented by the formula (4) : 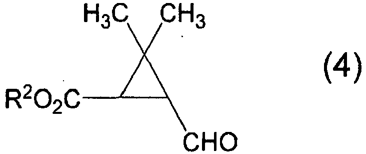 (wherein, R² is as defined above.)
and a compound represented by the formula (5): 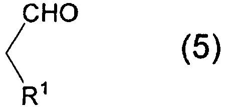 (wherein, R¹ is as defined above.)
are condensed in the presence of a base, and other methods, are mentioned.

Next, the oximation step will be explained.

In the oximation step, a formyl compound (1) and hydroxylamine are reacted in the presence of at least one compound selected from the group consisting of carboxylic acids and carboxylic acid metal salts, for example, in the presence of a carboxylic acid or a carboxylic acid metal salt, thereby the formyl compound (1) is converted into a hydroxyiminoalkenylcyclopropanecarboxylic acid ester represented by the formula (3) (hereinafter, referred to as hydroxyimino compound (3) in some cases.).

The carboxylic acid used in the oximation step includes, for example, carboxylic acids having 3 or more carbon atoms such as propanoic acid, butanoic acid, pentanoic acid, hexanoic acid, heptanoic acid, octanoic acid, 2-ethylhexanoic acid, nonanoic acid, decanoic acid, myristic acid and the like. As the carboxylic acid, carboxylic acids having 6 or more carbon atoms such as hexanoic acid, heptanoic acid, octanoic acid, 2-ethylhexanoic acid, nonanoic acid, decanoic acid, myristic acid and the like are preferable, and carboxylic acids having 6 or more and 14 or less carbon atoms are more preferable, for obtaining the hydroxyimino compound (3) with high yield. In the case of reacting the formyl compound (1) and hydroxylamine before removal of a carboxylic acid or in the case of reacting the hydroxyimino compound (3) and a dehydrating agent before removal of a carboxylic acid in the nitrilation step described later, carboxylic acids having 6 to 8 carbon atoms such as hexanoic acid, heptanoic acid, octanoic acid, 2-ethylhexanoic acid and the like are more preferable, linear carboxylic acids having 6 to 8 carbon atoms such as hexanoic acid, heptanoic acid, octanoic acid and the like are further preferable, and octanoic acid is still further preferable, since the carboxylic acid can be removed more easily.

The carboxylic acid metal salt includes alkali metal salts such as potassium salts, sodium salts, lithium salts and the like of the above-described carboxylic acids having 3 or more carbon atoms. Such a carboxylic acid metal salt may be generated in the reaction system of the oximation step by reacting the above-described carboxylic acid with an alkali metal hydroxide such as potassium hydroxide, sodium hydroxide, lithium hydroxide and the like or an alkali metal carbonate such as potassium carbonate, sodium carbonate, lithium carbonate and the like.

The use amount of the carboxylic acid or the carboxylic acid metal salt is preferably in the range of 0.01 to 0.5 mol, more preferably in the range of 0.05 to 0.2 mol, in total, with respect to 1 mol of the formyl compound (1).

The use amount of hydroxylamine used in the oximation step is preferably in the range of 0.8 to 2 mol, more preferably in the range of 1.0 to 1.3 mol, with respect to 1 mol of the formyl compound (1).

Hydroxylamine is preferably used in the form of an aqueous solution. Hydroxylamine may form a salt such as a hydrochloride, a hydrosulfate and the like, and when hydroxylamine forms a salt, it is preferable that hydroxylamine is liberated by contacting with an inorganic base, and the like. Examples of the inorganic base include alkali metal hydroxides such as potassium hydroxide, sodium hydroxide, lithium hydroxide and the like, and alkali metal carbonates such as potassium carbonate, sodium carbonate, lithium carbonate and the like. The inorganic base is preferably used in the form of an aqueous solution.

The use amount of the inorganic base is preferably in the range of 0.9 to 2.5 mol, more preferably in the range of 1.0 to 1.4 mol, with respect to 1 mol of the formyl compound (1).

In the oximation step, the reaction of the formyl compound (1) and hydroxylamine is carried out in a solvent. Examples of such a solvent include aromatic hydrocarbon solvents such as toluene, xylene, ethylbenzene and the like; chain hydrocarbon solvents such as n-pentane, n-hexane and the like; cyclic hydrocarbon solvents such as cyclopentane, cyclohexane and the like; halogenated aromatic hydrocarbon solvents such as monochlorobenzene and the like; ether solvents such as diethyl ether, tert-butyl methyl ether, 1,2-dimethoxyethane, 1,4-dioxane, tetrahydrofuran, anisole and the like; alcohol solvents such as methanol, ethanol, isopropyl alcohol and the like; and water, and a mixed solvent composed of water and an organic solvent immiscible with water (hereinafter, "organic solvent immiscible with water" is referred to as "immiscible organic solvent".) is preferable.

Examples of the immiscible organic solvent include aromatic hydrocarbon solvents such as toluene, xylene, ethylbenzene and the like; chain hydrocarbon solvents such as n-pentane, n-hexane and the like; cyclic hydrocarbon solvents such as cyclopentane, cyclohexane and the like; halogenated aromatic hydrocarbon solvents such as monochlorobenzene and the like; and ether solvents such as diethyl ether, tert-butyl methyl ether, 1,2-dimethoxyethane, 1,4-dioxane, tetrahydrofuran, anisole and the like.

The use amount of the solvent is preferably in the range of 1 to 40 parts by weight with respect to 1 part by weight of the formyl compound (1). When a mixture composed of water and the immiscible organic solvent is used as the solvent, the use amount of water is preferably in the range of 0.5 to 20 parts by weight and the use amount of the immiscible organic solvent is preferably in the range of 0.5 to 20 parts by weight, with respect to 1 part by weight of the formyl compound (1).

The mixing order in the oximation step is not restricted, and there are, for example, a method in which the formyl compound (1) and a carboxylic acid or a carboxylic acid metal salt are mixed, in a solvent, and hydroxylamine is added to the resultant mixture, and a method in which the formyl compound (1) and hydroxylamine are mixed, in a solvent, and a carboxylic acid or a carboxylic acid metal salt is added to the resultant mixture.

When hydroxylamine forms a salt, a method in which the formyl compound (1), a salt of hydroxylamine, and a carboxylic acid or a carboxylic acid metal salt are mixed, in a solvent, and an inorganic base is added to the resultant mixture and a method in which the formyl compound (1) and a carboxylic acid or a carboxylic acid metal salt are mixed, in a solvent, and an inorganic base is added to the resultant mixture while adding a salt of hydroxylamine, are preferable.

In the method of adding an inorganic base while adding a salt of hydroxylamine, it is more preferable that the addition time of a salt of hydroxylamine and the addition time of an inorganic base are approximately identical. Here, that the addition time of a salt of hydroxylamine and the addition time of an inorganic base are approximately identical means that the addition time of an inorganic base is, for example, in the range of 0.8 to 1.2-fold of the addition time of a salt of hydroxylamine. The addition time of a salt of hydroxylamine and the addition time of an inorganic base are, for example, in the range of 1 to 30 hours, preferably in the range of 3 to 10 hours.

In the oximation step, it is preferable that pH of the resultant mixture is kept at 7.5 or less. As the method for keeping pH at 7.5 or less, there is, for example, a method in which 10 to 20% of a salt of hydroxylamine to be added is previously mixed with the formyl compound (1) and a carboxylic acid, and an inorganic base is added to this while adding the salt of hydroxylamine.

The oximation step needs no heating, and the hydroxyimino compound (3) can be obtained with practical yield even if carried out at ambient temperature (about 20°C). Depending on the desired reaction time, the reaction temperature can also be controlled in a range in which the energy consumption amount can be suppressed low. The reaction temperature is, for example in the range of 0 to 60°C, preferably in the range of 5 to 50°C, more preferably in the range of 10 to 35°C.

In the oximation step, the reaction time excluding the above-described addition time is, for example in the range of 1 minute to 40 hours, preferably in the range of 10 minutes to 24 hours, further preferably in the range of 30 minutes to 10 hours, still further preferably in the range of 1 hour to 6 hours.

The degree of progress of the reaction in the oximation step can be confirmed by analysis means such as gas chromatography, high performance liquid chromatography and the like.

The reaction mixture obtained after the reaction contains a carboxylic acid or a carboxylic acid metal salt. The carboxylic acid or the carboxylic acid metal salt contained in the reaction mixture can be removed into an aqueous solution of pH 10 or more by washing the reaction mixture with an alkali aqueous solution and the like, and when the hydroxyimino compound (3) is converted into a cyanoalkenylcyclopropanecarboxylic acid ester represented by the formula (2) (hereinafter, referred to as cyano compound (2) in some cases.), it is particularly preferable that the carboxylic acid or the carboxylic acid metal salt is removed after reacting the hydroxyimino compound (3) and a dehydrating agent in the nitrilation step described later, from the standpoint of improvement in the yield of the cyano compound (2).

The reaction mixture can be subjected to a post treatment such as water-washing and the like, then, dehydrated if necessary, or the reaction mixture can be subjected to a post treatment such as neutralization, extraction, water-washing and the like, then, subjected to purification such as crystallization, extraction, distillation, adsorption on activated carbon, silica, alumina and the like, chromatographic purification such as silica gel column chromatography, and the like.

As thus obtainable hydroxyimino compound (3), compounds described in Tables 4, 5 and 6 are specifically exemplified.

**Table 4**

| | | | | | | | | |
|---|---|---|---|---|---|---|---|---|
| 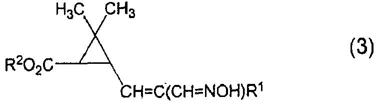 | | | | | | | | |

| Number | R¹ | R² | Number | R¹ | R² | Number | R¹ | R² |
|---|---|---|---|---|---|---|---|---|
| (3-1) | -CH₃ | -CH₃ | (3-21) | -CH₃ | -CH₂CH₃ | (3-41) | -CH₃ | 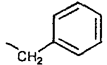 |
| (3-2) | -CH₂CH₃ | -CH₃ | (3-22) | -CH₂CH₃ | -CH₂CH₃ | (3-42) | -CH₂CH₃ | 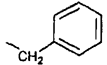 |
| (3-3) | -CH₂CH₂CH₃ | -CH₃ | (3-23) | -CH₂CH₂CH₃ | -CH₂CH₃ | (3-43) | -CH₂CH₂CH₃ | 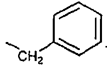 |
| (3-4) | 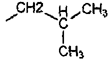 | -CH₃ | (3-24) | 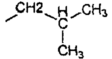 | -CH₂CH₃ | (3-44) | 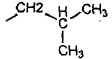 | 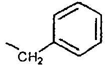 |
| (3-5) | 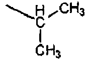 | -CH₃ | (3-25) | 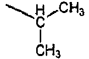 | -CH₂CH₃ | (3-45) | 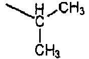 | 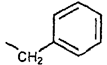 |
| (3-6) | 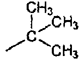 | -CH₃ | (3-26) | 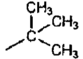 | -CH₂CH₃ | (3-46) | 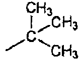 | 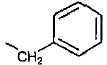 |
| (3-7) | -(CH₂)₄CH₃ | -CH₃ | (3-27) | -(CH₂)₄CH₃ | -CH₂CH₃ | (3-47) | -(CH₂)₄CH₃ | 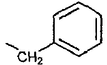 |
| (3-8) | 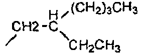 | -CH₃ ' | (3-28) | 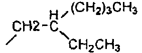 | -CH₂CH₃ | (3-48) | 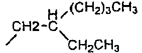 | 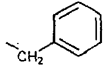 |
| (3-9) | 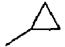 | -CH₃ | (3-29) | 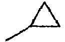 | -CH₂CH₃ | (3-49) | 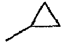 | 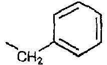 |
| (3-10) | 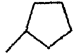 | -CH₃ | (3-30) | 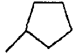 | -CH₂CH₃ | (3-50) | 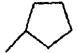 | 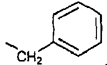 |
| (3-11) | 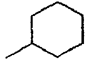 | -CH₃ | (3-31) | 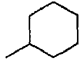 | -CH₂CH₃ | (3-51) | 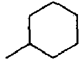 | 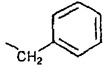 |
| (3-12) | -F | -CH₃ | (3-32) | -F | -CH₂CH₃ | (3-52) | -F | 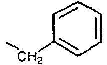 |
| (3-13) | -Cl | -CH₃ | (3-33) | -Cl | -CH₂CH₃ | (3-53) | -Cl | 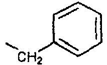 |
| (3-14) | -Br | -CH₃ | (3-34) | -Br | -CH₂CH₃ | (3-54) | -Br | 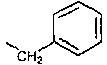 |
| (3-15) | -I | -CH₃ | (3-35) | -I | -CH₂CH₃ | (3-55) | -I | 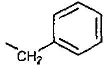 |

**Table 5**

| | | | | | | | | |
|---|---|---|---|---|---|---|---|---|
| 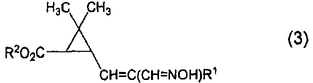 | | | | | | | | |

| Number | R¹ | R² | | Number R¹ | R² | Number | R¹ | R² |
|---|---|---|---|---|---|---|---|---|
| (3-61) | -CH₃ | 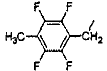 | (3-81) | -CH₃ | 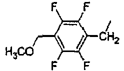 | (3-101) | -CH₃ | 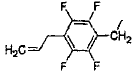 |
| (3-62) | -CH₂CH₃ | 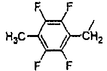 | (3-82) | -CH₂CH₃ | 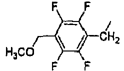 | (3-102) | -CH₂CH₃ | 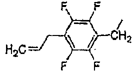 |
| (3-63) | -CH₂CH₂CH₃ | 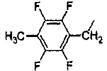 | (3-83) | -CH₂CH₂CH₃ | 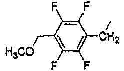 | (3-103) | -CH₂CH₂CH₃ | 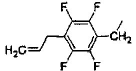 |
| (3-84) | 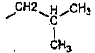 | 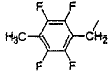 | (3-84) | 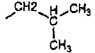 | 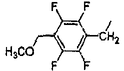 | (3-104) | 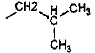 | 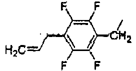 |
| (3-65) | 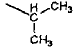 | 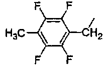 | (3-85) | 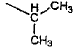 | 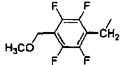 | (3-105) | 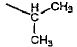 | 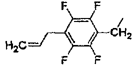 |
| (3-66) | 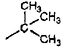 | 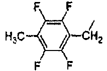 | (3-86) | 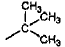 | 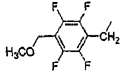 | (3-106) | 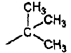 | 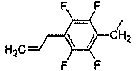 |
| (3-67) | -(CH₂)₄CH₃ | 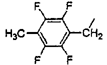 | (3-87) | -(CH₂)₄CH₃ | 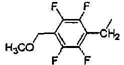 | (3-107) | -(CH₂)₄CH₃ | 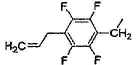 |
| (3-68) | 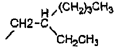 | 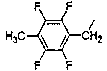 | (3-88) | 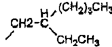 | 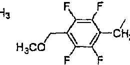 | (3-108) | 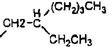 | 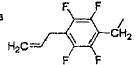 |
| (3-69) | 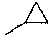 | 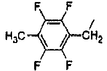 | (3-89) | 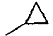 | 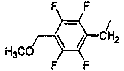 | (3-109) | 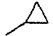 | 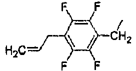 |
| (3-70) | 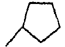 | 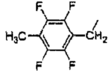 | (3-90) | 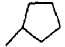 | 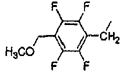 | (3-110) | 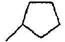 | 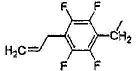 |
| (3-71) | 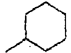 | 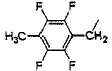 | (3-91) | 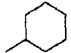 | 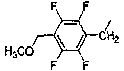 | (3-111) | 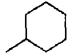 | 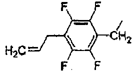 |
| (3-72) | -F | 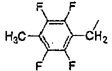 | (3-92) | -F | 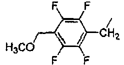 | (3-112) | -F | 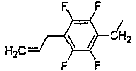 |
| (3-73) | -Cl | 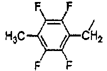 | (3-93) | -Cl | 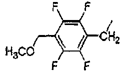 | (3-113) | -Cl | 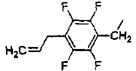 |
| (3-74) | -Br | 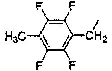 | (3-94) | -Br | 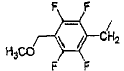 | (3-114) | -Br | 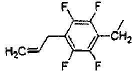 |
| (3-75) | -I | 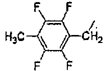 | (3-95) | -I | 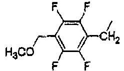 | (3-115) | -I | 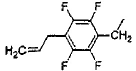 |

**Table 6**

| | | | | | |
|---|---|---|---|---|---|
| 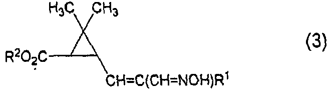 | | | | | |

| Number | R¹ | R² | Number | R¹ | R² |
|---|---|---|---|---|---|
| (3-116) | -CH₃ | 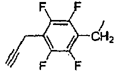 | (3-131) | -CH₃ | 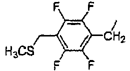 |
| (3-117) | -CH₂CH₃ | 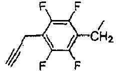 | (3-132) | -CH₂CH₃ | 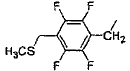 |
| (3-118) | -CH₂CH₂CH₃ | 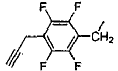 | (3-133) | -CH₂CH₂CH₃ | 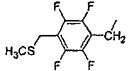 |
| (3-119) | 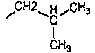 | 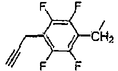 | (3-134) | 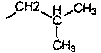 | 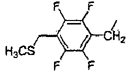 |
| (3-120) | 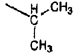 | 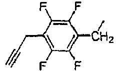 | (3-135) | 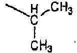 | 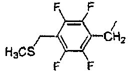 |
| (3-121) | 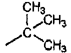 | 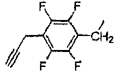 | (3-136) | 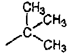 | 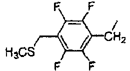 |
| (3-122) | -(CH₂)₄CH₃ | 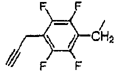 | (3-137) | -(CH₂)₄CH₃ | 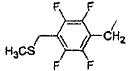 |
| (3-123) | 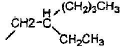 | 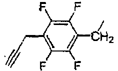 | (3-138) | 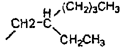 | 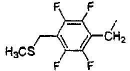 |
| (3-124) | 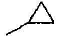 | 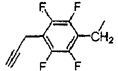 | (3-139) | 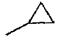 | 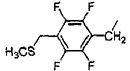 |
| (3-125) | 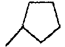 | 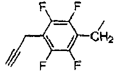 | (3-140) | 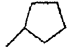 | 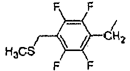 |
| (3-126) | 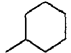 | 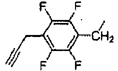 | (3-141) | 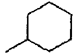 | 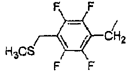 |
| (3-127) | -F | 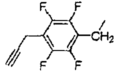 | (3-142) | -F | 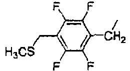 |
| (3-128) | -Cl | 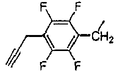 | (3-143) | -Cl | 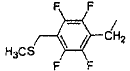 |
| (3-129) | -Br | 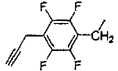 | (3-144) | -Br | 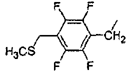 |
| (3-130) | -I | 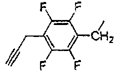 | (3-145) | -I | 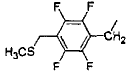 |

As the hydroxyimino compound (3), compounds represented by the number (3-1), (3-13), (3-21), (3-33), (3-61), (3-73), (3-81) or (3-93) in the tables are preferable, compounds represented by the number (3-1), (3-21), (3-61) or (3-81) in the tables are more preferable.

Next, the nitrilation step will be explained.

In the nitrilation step, the hydroxyimino compound (3) and a dehydrating agent are reacted, thereby the hydroxyimino compound (3) is converted into a cyano compound (2).

In the present specification, the dehydrating agent means a compound showing an action of detaching a hydroxyl group and hydrogen of -CH=NOH in the hydroxyimino compound (3), thereby converting -CH=NOH into -CN.

The above-described dehydrating agent includes carboxylic anhydrides such as acetic anhydride and the like; carboxylic halides; sulfonic halides; thionyl halides, and the like.

The carboxylic halide (referred to also as "acyl halide") includes acetyl halides such as acetyl chloride, acetyl bromide and the like; propionyl halides such as propionyl chloride, propionyl bromide and the like; butyroyl halides such as butyroyl chloride, butyroyl bromide and the like; etc.

The sulfonic halide includes methanesulfonyl halides such as methanesulfonyl chloride, methanesulfonyl bromide and the like; etc.

The thionyl halide includes thionyl chloride, thionyl bromide and the like.

The carboxylic anhydride includes carboxylic anhydrides having 2 to 8 carbon atoms, and the like, specifically, acetic anhydride, propionic anhydride, butanoic anhydride and the like. The carboxylic anhydride is preferably acetic anhydride.

Of these dehydrating agents, carboxylic halides, sulfonic halides and thionyl halides generate an acid in reacting with the hydroxyimino compound (3).

In the nitrilation step, one dehydrating agent may be used or two or more dehydrating agents may be used.

Such a dehydrating agent is preferably at least one compound selected from the group consisting of carboxylic anhydrides and carboxylic halides.

When two or more such dehydrating agents are used, it is preferable to use a carboxylic anhydride as one dehydrating agent, it is more preferable to use a carboxylic anhydride, and at least one compound selected from the group consisting of carboxylic halides, sulfonic halides and thionyl halides, and it is further preferable to use a carboxylic anhydride, and at least one compound selected from the group consisting of acetyl chloride, acetyl bromide, thionyl chloride and methanesulfonyl chloride.

The use amount of the dehydrating agent is preferably in the range of 0.8 to 3.0 mol, more preferably in the range of 1.0 to 2.0 mol, in total, with respect to 1 mol of the hydroxyimino compound (3).

When a carboxylic anhydride is used as the dehydrating agent, the use amount of the carboxylic anhydride is preferably in the range of 0.8 to 2 mol, more preferably in the range of 1.0 to 1.5 mol, with respect to 1 mol of the hydroxyimino compound (3).

It is preferable that the nitrilation step is carried out in the presence of at least one acid selected from the group consisting of hydrogen halides and organic sulfonic acids, since the reaction is promoted. Details of the acid will be described later.

When a compound selected from the group consisting of carboxylic halides, sulfonic halides and thionyl halides is used as a dehydrating agent in the nitrilation step as described above, the nitrilation step can be carried out in the presence of the above-described acid. Therefore, when two or more dehydrating agents are used, it is preferable to use any of a carboxylic halide, a sulfonic halide and a thionyl halide as one dehydrating agent.

In the nitrilation step, the reaction of the hydroxyimino compound (3) and a dehydrating agent is preferably carried out in the presence of an organic base.

The organic base includes, for example, aromatic amines such as pyridine, methylethylpyridine, dimethylaminopyridine and the like, and aliphatic tertiary amines such as triethylamine and the like. The organic base is preferably pyridine.

The organic base may also be a compound forming a salt with at least one acid selected from the group consisting of hydrogen halides and organic sulfonic acids.

The hydrogen halide includes hydrochloric acid, hydrobromic acid, hydroiodic acid and the like. The organic sulfonic acid includes aliphatic sulfonic acids such as alkylsulfonic acids having 1 to 4 carbon atoms and the like; and aromatic sulfonic acids such as aromatic hydrocarbonsulfonic acids having 6 to 10 carbon atoms and the like.

The aliphatic sulfonic acid includes methanesulfonic acid, ethanesulfonic acid and propanesulfonic acid. The aromatic sulfonic acid includes organic sulfonic acids such as benzenesulfonic acid, toluenesulfonic acid and the like.

The salt of the organic base with the above-described acid includes hydrochlorides of aromatic amines, hydrochlorides of aliphatic tertiary amines, hydrobromides of aromatic amines, hydroiodides of aromatic amines, hydrochlorides of aliphatic tertiary amines, alkylsulfonic acid salts of aromatic amines; and the like. Such salts include, specifically, pyridinehydrochloride, methylethylpyridinehydrochloride, dimethylaminopyridinehydrochloride, triethylaminehydrochloride, pyridinehydrobromide, pyridinehydroiodide, pyridinemethanesulfonic acid salt, pyridineethanesulfonic acid salt, pyridinepropanesulfonic acid salt, pyridinebenzenesulfonic acid salt and pyridinetoluenesulfonic acid salt, and preferable is pyridinehydrochloride or pyridinehydrobromide, more preferable is pyridinehydrochloride.

The nitrilation step is preferably carried out in the presence of the organic base, and a salt of the organic base with the above-described acid.

The use amount of the organic base is preferably in the range of 0.8 to 2 mol, more preferably in the range of 1.0 to 1.5 mol, with respect to 1 mol of the hydroxyimino compound (3). The use amount of the organic base is, in the case of use of a salt of the organic base with the above-described acid, the total amount including also the amount of the base derived from the salt.

In the nitrilation step, the reaction of the hydroxyimino compound (3) and a dehydrating agent is preferably carried out in the presence of a solvent. Such a solvent includes, for example, immiscible organic solvents exemplified in the oximation step. In the case of use of an immiscible organic solvent in the oximation step, the immiscible organic solvent can be used as it is in the form of a mixture with the hydroxyimino compound (3), or if necessary, can be diluted with a solvent and used. The use amount of the solvent is usually in the range of 0 to 20 parts by weight, preferably in the range of 0.5 to 20 parts by weight, more preferably in the range of 0.5 to 5 parts by weight, with respect to 1 part by weight of the hydroxyimino compound (3).

The mixing order in the nitrilation step is not restricted, and there are, for example, a method in which a solvent and an organic base are mixed, and the hydroxyimino compound (3) and a dehydrating agent are added to this, and a method in which a solvent, an organic base and the hydroxyimino compound (3) are mixed, and a dehydrating agent is added to this, and preferable is a method in which a solvent and an organic base are mixed, and the hydroxyimino compound (3) and a dehydrating agent are added to this.

In this preferable method, it is preferable to add a dehydrating agent to a mixture of a solvent and an organic base while adding the hydroxyimino compound (3). In the case of adding a dehydrating agent to a mixture of a solvent and an organic base while adding the hydroxyimino compound (3), it is more preferable that the addition time of the hydroxyimino compound (3) and the addition time of a dehydrating agent are approximately identical.

The amount of a solvent in a mixture of a solvent and an organic base is preferably in the range of 0 to 10 parts by weight, more preferably in the range of 0.5 to 2 parts by weight, with respect to 1 part by weight of the hydroxyimino compound (3). Here, that the addition time of the hydroxyimino compound (3) and the addition time of a dehydrating agent are approximately identical means that the addition time of a dehydrating agent is in the range of 0.8 to 1.2-fold of the addition time of the hydroxyimino compound (3).

In this preferable method, the addition time of the hydroxyimino compound (3) is usually in the range of 1 to 30 hours, preferably in the range of 3 to 10 hours.

The reaction temperature in the nitrilation step is preferably in the range of 80 to 150°C, more preferably in the range of 90 to 110°C.

In the nitrilation step, the reaction time excluding the above-described addition time is for example in the range of 1 minute to 24 hours, preferably in the range of 1 hour to 10 hours, further preferably in the range of 1 to 5 hours.

Preferable embodiments of the nitrilation step include a step of reacting the hydroxyimino compound (3) and a carboxylic anhydride in the presence of an organic base and at least one acid selected from the group consisting of hydrogen halides and sulfonic acids.

In this embodiment, as the carboxylic anhydride and the organic base, those listed above respectively are preferably mentioned. The carboxylic anhydride is used as a dehydrating agent.

In this embodiment, the acid is selected from the group consisting of hydrogen halides and organic sulfonic acids.

The above-described hydrogen halide includes hydrogen chloride, hydrogen bromide, hydrogen iodide and the like.

The above-described organic sulfonic acid includes aliphatic sulfonic acids such as methanesulfonic acid, ethanesulfonic acid, propanesulfonic acid and the like; and aromatic sulfonic acids such as benzenesulfonic acid, toluenesulfonic acid and the like.

It is more preferable that the above-described acid is a hydrogen halide. The hydrogen halide is preferably hydrogen chloride or hydrogen bromide.

The above-described acid may be a commercially marketed product, may be generated by any known method, or may be generated in the reaction system of the nitrilation step.

The method of generating the above-described acid in the reaction system of the nitrilation step includes, for example, a method in which a compound generating the above-described acid by contact with the hydroxyimino compound (3) (this compound is referred to as "acid generator" in some cases.) is used in the reaction of the hydroxyimino compound (3) and a carboxylic anhydride, instead of the above-described acid or together with the above-described acid.

The acid generator includes the following compounds.
- compounds selected from the group consisting of carboxylic halides, sulfonic halides and thionyl halides
- metal halides
- boron halides

The compound selected from the group consisting of carboxylic halides, sulfonic halides and thionyl halides shows an action, as a dehydrating agent, of converting - CH=NOH of the hydroxyimino compound (3) into -CN, as described above, together with an action of generating the above-described acid. Therefore, when a carboxylic anhydride, and a compound selected from the group consisting of carboxylic halides and sulfonic halides are used together, as a dehydrating agent, then, nitrilation of the hydroxyimino compound (3) can be carried out efficiently.

The metal halide includes aluminum halides such as aluminum chloride, aluminum bromide and the like; and zirconium halides such as zirconium chloride, zirconium bromide and the like.

The boron halide includes boron halides such as boron chloride, boron bromide and the like.

The acid generated in the reaction system of the nitrilation step is preferably an acid generated from at least one compound selected from the group consisting of carboxylic halides, organic sulfonic halides, metal halides and thionyl halides, more preferably an acid generated from at least one compound selected from the group consisting of acetyl halides and aluminum halides.

The above-described acid and the acid generator can be used singly, or can be mixed with the above-described solvent and acetic acid and the like.

The use amount of the above-described acid is preferably in the range of 0.05 to 1 mol, more preferably in the range of 0.1 to 0.5 mol, with respect to 1 mol of the hydroxyimino compound (3). The use amount of the acid is, in the case of use of an acid generator and a salt of an organic base with the above-described acid, the total amount including also the amount of the acid derived from the acid generator and the salt.

When the above-described acid is generated from an acid generator in the reaction system of the nitrilation step, the use amount of the acid generator varies depending on the mol number of the acid generatable from 1 mol of the acid generator, and for example, if 1 mol of the acid can be generated from 1 mol of the acid generator, the use amount is preferably in the range of 0.05 to 1 mol, more preferably in the range of 0.1 to 0.5 mol, with respect to 1 mol of the hydroxyimino compound (3).

In the nitrilation step, the mixing method of reaction reagents is not limited, and preferable is
(a) a method in which a carboxylic anhydride, an organic base and an acid or an acid generator are mixed, and the hydroxyimino compound (3) is added to the resultant mixture,
(b) a method in which the hydroxyimino compound (3), an organic base and an acid or an acid generator are mixed, and a carboxylic anhydride is added to the resultant mixture, or
(c) a method in which an organic base and an acid or an acid generator are mixed, and the hydroxyimino compound (3) and a carboxylic anhydride are added to the resultant mixture.

In preparing the mixture described in (a) to (c), it is preferable that a solvent is further mixed. In the method described in (a), the hydroxyimino compound (3) may be mixed with a solvent and added, in the method described in (b), a carboxylic anhydride may be mixed with a solvent and added, and in the method described in (c), the hydroxyimino compound (3) and/or a carboxylic anhydride may be mixed with a solvent and added.

The method described in (c) is more preferable, and in the method described in (c), it is further preferable that the hydroxyimino compound (3) is mixed with a solvent and added.

The addition time of the hydroxyimino compound (3) to the mixture described in (a), the addition time of a carboxylic anhydride to the mixture described in (b) and the addition time of the hydroxyimino compound (3) and a carboxylic anhydride to the mixture described in (c) are preferably 1 hour or more, more preferably 3 hours or more, from the standpoint of successive conversion of a reaction intermediate represented by the formula (3-1): 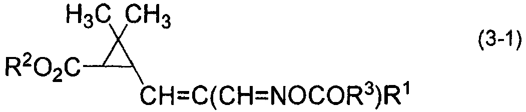 (wherein, R¹ and R² are as defined above. R³ represents a group derived from a carboxylic anhydride.)
generated from the hydroxyimino compound (3) into a cyano compound (2). From the standpoint of productivity, 30 hours or less are preferable, 10 hours or less are more preferable.

Here, the addition time means a time necessary from initiation of addition of the hydroxyimino compound (3) to the mixture described in (a) to termination thereof, a time necessary from initiation of addition of a carboxylic anhydride to the mixture described in (b) to termination thereof, or a time necessary from initiation of addition of the hydroxyimino compound (3) to the mixture described in (c) to termination thereof, and a time necessary from initiation of addition of a carboxylic anhydride to the mixture described in (c) to termination thereof.

In the case of effecting the method described in (c), it is preferable that the addition time of the hydroxyimino compound (3) and the addition time of a carboxylic anhydride are approximately identical.

Here, that the addition time of the hydroxyimino compound (3) and the addition time of a carboxylic anhydride are approximately identical means that the addition time of a carboxylic anhydride is, for example, in the range of 0.8 to 1.2-fold of the addition time of the hydroxyimino compound (3). The addition time of a carboxylic anhydride is preferably in the range of 0.9 to 1.1-fold, more preferably in the range of 0.95 to 1.05-fold of the addition time of the hydroxyimino compound (3). It is further preferable that addition of the hydroxyimino compound (3) and addition of a carboxylic anhydride are started simultaneously and terminated simultaneously.

The degree of progress of the reaction in the nitrilation step can be confirmed by analysis means such as gas chromatography, high performance liquid chromatography and the like.

When the reaction is carried out in the presence of water and an immiscible solvent, a cyano compound (2) can be taken out by, for example, mixing the reaction mixture obtained after the reaction with water, performing a post treatment such as extraction, water-washing, filtration and the like, then, performing isolation such as distillation, crystallization and the like.

The immiscible solvent includes, for example, the aromatic hydrocarbon solvents, saturated chain hydrocarbon solvents, alicyclic hydrocarbon solvents and halogenated aromatic hydrocarbon solvents described above.

When the reaction is carried out in the presence of a solvent miscible with water or in the absence of a solvent, a cyano compound (2) can be taken out by, for example, mixing the reaction mixture obtained after the reaction, the above-described immiscible solvent and water, performing a post treatment such as extraction, water-washing, filtration and the like, then, performing isolation such as distillation, crystallization and the like.

When the reaction mixture contains an organic base, this can be removed from the reaction mixture by washing the reaction mixture with an aqueous solution of a strong acid such as sulfuric acid, hydrochloric acid, methanesulfonic acid and the like in a post treatment. The use amount of the strong acid used for washing is preferably 0.5 mol or more in the case of a monobasic acid, preferably 0.25 mol or more in the case of a dibasic acid, with respect to 1 mol of the organic base.

When the reaction mixture contains a formyl compound (1), the formyl compound (1) can be removed from the reaction mixture by washing the reaction mixture with an alkali aqueous solution such as a potassium hydroxide aqueous solution, a sodium hydroxide aqueous solution, a lithium hydroxide aqueous solution, a potassium carbonate aqueous solution, a sodium carbonate aqueous solution and the like and/or water, in a post treatment.

The reaction mixture obtained after the reaction contains a carboxylic acid or a carboxylic acid metal salt used in the oximation step in some cases.

When the reaction mixture contains a carboxylic acid or a carboxylic acid metal salt, the carboxylic acid or the carboxylic acid metal salt can be removed into an alkali aqueous solution by washing the reaction mixture with an aqueous solution of a strong acid such as sulfuric acid, hydrochloric acid and the like, then, washing with an alkali aqueous solution, or washing the reaction mixture as it is with an alkali aqueous solution. It is preferable that pH of the alkali aqueous solution after washing is 10 or more, and such an alkali aqueous solution includes alkali metal hydroxide aqueous solutions such as a potassium hydroxide aqueous solution, a sodium hydroxide aqueous solution, a lithium hydroxide aqueous solution and the liKe, and alkali metal carbonate aqueous solutions such as a potassium carbonate aqueous solution, a sodium carbonate aqueous solution and the like.

The concentration of the alkali aqueous solution is for example in the range of 0.1 to 30 wt%, preferably in the range of 5 to 15 wt%. The use amount of the alkali aqueous solution is in the range of 0.1 to 2 mol, preferably in the range of 0.4 to 1.0 mol with respect to 1 mol of the hydroxyimino compound (3). It is preferable that after washing with the alkali aqueous solution, water-washing is further performed.

The resultant cyano compound (2) can be purified by, for example, recrystallization; extraction; distillation; treatment of adsorption on activated carbon, silica, alumina and the like; chromatographic methods such as silica gel column chromatography and the like; etc.

As thus obtainable cyano compound (2), compounds described in Tables 7, 8 and 9 are specifically exemplified.

**Table 7**

| | | | | | | | | |
|---|---|---|---|---|---|---|---|---|
| 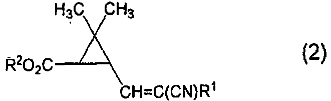 | | | | | | | | |

| Number | R¹ | R² | Number | R¹ | R² | Number | R¹ | R² |
|---|---|---|---|---|---|---|---|---|
| (2-1) | -CH₃ | -CH₃ | (2-21) | -CH₃ | -CH₂CH₃ | (2-41) | -CH₃ | 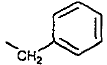 |
| (2-2) | -CH₂CH₃ | -CH₃ | (2-22) | -CH₂CH₃ | -CH₂CH₃ | (2-42) | -CH₂CH₃ | 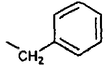 |
| (2-3) | -CH₂CH₂CH₃ | -CH₃ | (2-23) | -CH₂CH₂CH₃ | -CH₂CH₃ | (2-43) | -CH₂CH₂CH₃ | 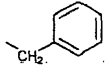 |
| (2-4) | 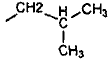 | -CH₃ | (2-24) | 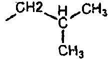 | -CH₂CH₃ | (2-44) | 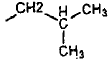 | 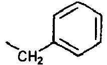 |
| (2-5) | 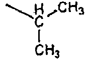 | -CH₃ | (2-25) | 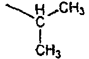 | -CH₂CH₃ | (2-45) | 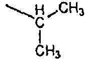 | 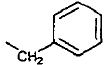 |
| (2-6) | 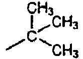 | -CH₃ | (2-26) | 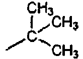 | -CH₂CH₃ | (2-46) | 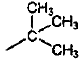 | 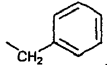 |
| (2-7) | -(CH₂)₄CH₃ | -CH₃ | (2-27) | -(CH₂)₄CH₃ | -CH₂CH₃ | (2-47) | -(CH₂)₄CH₃ | 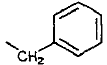 |
| (2-8) | 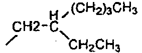 | -CH₃ | (2-28) | 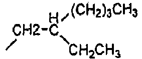 | -CH₂CH₃ | (2-48) | 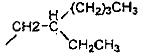 | 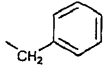 |
| (2-9) | 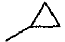 | -CH₃ | (2-29) | 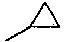 | -CH₂CH₃ | (2-49) | 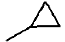 | 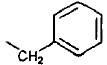 |
| (2-10) | 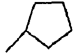 | -CH₃ | (2-30) | 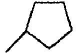 | -CH₂CH₃ | (2-50) | 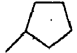 | 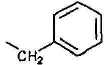 |
| (2-11) | 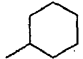 | -CH₃ | (2-31) | 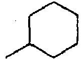 | -CH₂CH₃ | (2-51) | 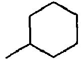 | 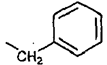 |
| (2-12) | -F | -CH₃ | (2-32) | -F | -CH₂CH₃ | (2-52) | -F | 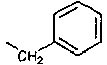 |
| (2-13) | -Cl | -CH₃ | (2-33) | -Cl | -CH₂CH₃ | (2-53) | -Cl | 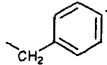 |
| (2-14) | -Br | -CH₃ | (2-34) | -Br | -CH₂CH₃ | (2-54) | -Br | 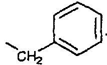 |
| (2-15) | -I | -CH₃ | (2-35) | -I | -CH₂CH₃ | (2-55) | -I | 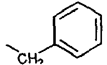 |

**Table 8**

| | | | | | | | | |
|---|---|---|---|---|---|---|---|---|
| 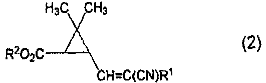 | | | | | | | | |

| Number | R¹ | R² | | Number R¹ | R² | Number | R¹ | R² |
|---|---|---|---|---|---|---|---|---|
| (2-61) | -CH₃ | 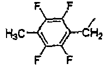 | (2-81) | -CH₃ | 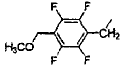 | (2-101) | -CH₃ | 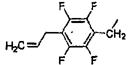 |
| (2-62) | -CH₂CH₃ | 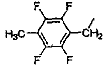 | (2-82) | -CH₂CH₃ | 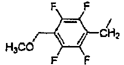 | (2-102) | -CH₂CH₃ | 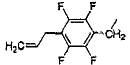 |
| (2-63) | -CH₂CH₂CH₃ | 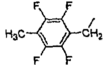 | (2-83) | -CH₂CH₂CH₃ | 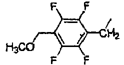 | (2-103) | -CH₃CH₂CH₃ | 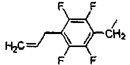 |
| (2-64) | 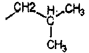 | 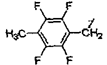 | (2-84) | 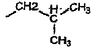 | 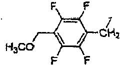 | (2-104) | 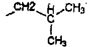 | 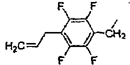 |
| (2-65) | 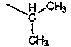 | 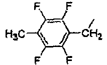 | (2-85) | 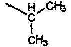 | 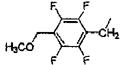 | (2-105) | 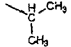 | 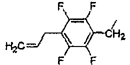 |
| (2-66) | 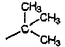 | 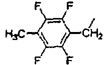 | (2-86) | 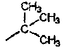 | 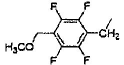 | (2-106) | 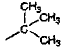 | 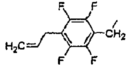 |
| (2-67) | -(CH₂)₄CH₃ | 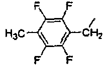 | (2-87) | -(CH₂)₄CH₃ | 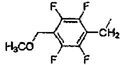 | (2-107) | -(CH₂)₄CH₃ | 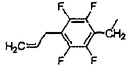 |
| (2-68) | 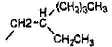 | 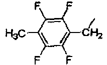 | (2-88) | 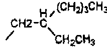 | 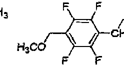 | (2-108) | 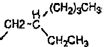 | 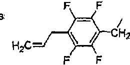 |
| (2-69) | 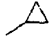 | 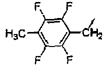 | (2-89) | 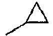 | 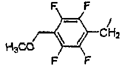 | (2-109) | 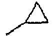 | 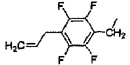 |
| (2-70) | 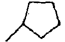 | 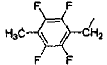 | (2-90) | 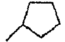 | 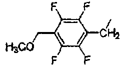 | (2-110) | 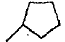 | 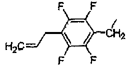 |
| (2-71) | 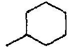 | 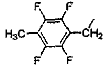 | (2-91) | 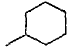 | 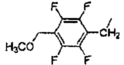 | (2-111) | 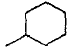 | 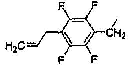 |
| (2-72) | -F | 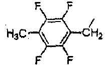 | (2-92) | -F | 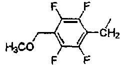 | (2-112) | -F | 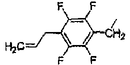 |
| (2-73) | -Cl | 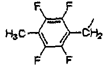 | (2-93) | -Cl | 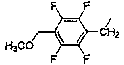 | (2-113) | -Cl | 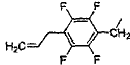 |
| (2-74) | -Br | 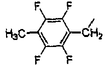 | (2-94) | -Br | 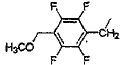 | (2-114) | -Br | 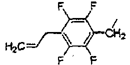 |
| (2-75) | -I | 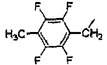 | (2-95) | -I | 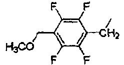 | (2-115) | -I | 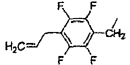 |

**Table 9**

| | | | | | |
|---|---|---|---|---|---|
| 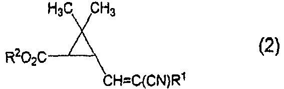 | | | | | |

| Number | R¹ | R² | Number | R¹ | R² |
|---|---|---|---|---|---|
| (2-116) | -CH₃ | 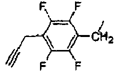 | (2-131) | -CH₃ | 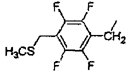 |
| (2-117) | -CH₂CH₃ | 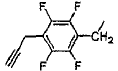 | (2-132) | -CH₂CH₃ | 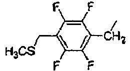 |
| (2-118) | -CH₂CH₂CH₃ | 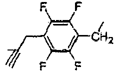 | (2-133) | -CH₂CH₂CH₃ | 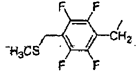 |
| (2-119) | 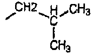 | 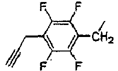 | (2-134) | 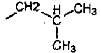 | 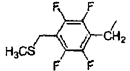 |
| (2-120) | 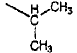 | 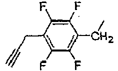 | (2-135) | 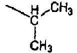 | 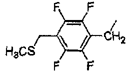 |
| (2-121) | 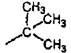 | 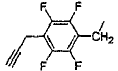 | (2-136) | 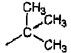 | 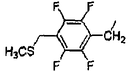 |
| (2-122) | -(CH₂)₄CH₃ | 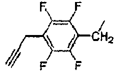 | (2-137) | -(CH₂)₄CH₃ | 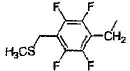 |
| (2-123) | 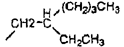 | 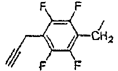 | (2-138) | 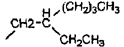 | 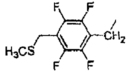 |
| (2-124) | 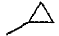 | 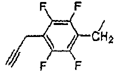 | (2-139) | 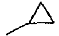 | 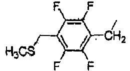 |
| (2-125) | 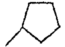 | 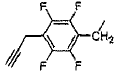 | (2-140) | 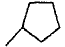 | 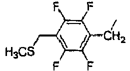 |
| (2-126) | 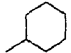 | 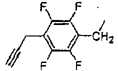 | (2-141) | 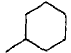 | 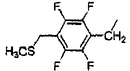 |
| (2-127) | -F | 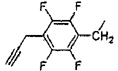 | (2-142) | -F | 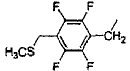 |
| (2-128) | -Cl | 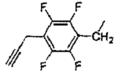 | (2-143) | -Cl | 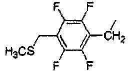 |
| (2-129) | -Br | 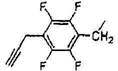 | (2-144) | -Br | 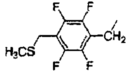 |
| (2-130) | -I | 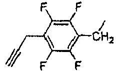 | (2-145) | -I | 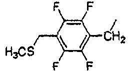 |

As the cyano compound (2), compounds represented by the number (2-1), (2-13), (2-21), (2-33), (2-61), (2-73), (2-81) or (2-93) in the tables are preferable, compounds represented by the number (2-1), (2-21), (2-61) or (2-81) in the tables are more preferable.

### EXAMPLES

The present invention will be described based on examples below.

The contents of the formyl compound (1), the hydroxyimino compound (3) and the cyano compound (2) were determined by a gas chromatography internal standard method using respective standard products of the formyl compound (1), the hydroxyimino compound (3) and the cyano compound (2) prepared separately by distillation, recrystallization and the like.

### <Example 1>

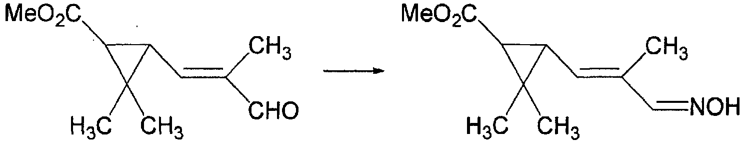

To 185.68 g of a mixed xylene solution of methyl trans-2,2-dimethyl-3-[(1E)-2-formyl-1-propenyl]cyclopropanecarboxylate (concentration: 47.54 wt%) were added 16.18 g of a hydroxylamine hydrosulfate aqueous solution (concentration: 24 wt%) and 6.63 g of octanoic acid (0.1 mol with respect to 1 mol of methyl trans-2,2-dimethyl-3-[(1E)-2-formyl-1-propenyl]cyclopropanecarboxylate). The resultant mixture showed pH2. Into this, 97.42 g of a 23% sodium hydroxide solution was dropped while dropping 160.89 g of a hydroxylamine hydrosulfate aqueous solution (concentration: 24 wt%) at 20°C. Both the dropping times were 4.5 hours. In completion of dropping, the resultant mixture showed pH6.5. An aqueous layer was separated from the reaction mixture obtained by thermally retaining at 20°C for 3 hours, the resultant oil layer was washed with water twice, then, pressure was reduced to 5 kPa, and dehydrated under reflux with heating to obtain 211.82 g of a mixed xylene solution of methyl trans-2,2-dimethyl-3-[(1E)-3-(hydroxyimino)-2-methyl-1-propenyl]cyclopropanecarboxylate (concentration: 42.73 wt%). Moisture content: 52 ppm, yield: 95.32%.

### <Example 2>

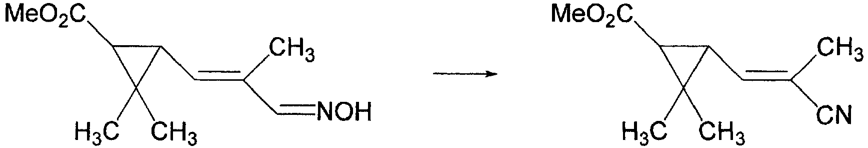

Under a nitrogen atmosphere, 10.35 g of pyridine, 21.24 g of mixed xylene and 2.41 g of acetyl chloride were mixed, the resultant mixture was heated up to 100°C. Into this, 10.40 g of acetic anhydride was dropped while dropping 52.63 g of a mixed xylene solution of methyl trans-2,2-dimethyl-3-[(1E)-3-(hydroxyimino)-2-methyl-1-propenyl]cyclopropanecarboxylate (concentration: 42.73 wt%). Both the dropping times were 6 hours. After completion of dropping, the resultant mixture was thermally retained for 2 hours, and cooled down to 20°C. To the cooled reaction mixture, 21.05 g of water and 5.60 g of 97 wt% sulfuric acid were added and mixed, and the mixture was liquid-separated to obtain an oil layer 1 and an aqueous layer 1. To the aqueous layer 1, 10.67 g of mixed xylene was added and mixed, and the mixture was liquid-separated to obtain an oil layer 2 which was then combined with the oil layer 1 obtained previously, to obtain an oil layer 3. To this, 21.12 g of water and 11.37 g of a 23 wt% sodium hydroxide aqueous solution were added and mixed, octanoic acid contained in the oil layer 3 was removed into an aqueous layer, then, the aqueous layer was liquid-separated to obtain an oil layer 4. The aqueous layer showed pH 12.3. To the resultant oil layer 4, 21.15 g of water was added and mixed, and the mixture was liquid-separated to obtain an oil layer which was then condensed, to obtain 24.45 g of a solid containing methyl trans-2,2-dimethyl-3-[(1E)-2-cyano-1-propen-1-yl]cyclopropanecarboxylate (content of methyl trans-2,2-dimethyl-3-[(1E)-2-cyano-1-propen-1-yl]cyclopropanecarboxylate: 82.34 wt%). Yield: 97.80%.

The amount of octanoic acid contained in the solid was 0.1 wt% or less with respect to methyl trans-2,2-dimethyl-3-[(1E)-2-cyano-1-propen-1-yl]cyclopropanecarboxylate.

### <Example 3>

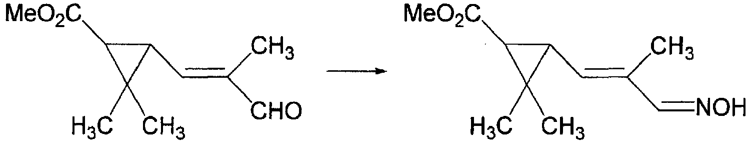

Under a nitrogen atmosphere, to 41.32 g of a mixed xylene solution of methyl trans-2,2-dimethyl-3-[(1E)-2-formyl-1-propenyl]cyclopropanecarboxylate as the formyl compound (1) (concentration: 47.54 wt%) were added 37.68 g of a hydroxylamine hydrosulfate aqueous solution (concentration: 24 wt%) and 1.48 g of octanoic acid as the carboxylic acid (10 mol% with respect to methyl trans-2,2-dimethyl-3-[(1E)-2-formyl-1-propenyl]cyclopropanecarboxylate), and into this, 22.16 g of a 23% sodium hydroxide solution was dropped at 20°C over a period of 5.5 hours. The mixture was thermally retained at 20°C for 1.5 hours, then, an aqueous layer was liquid-separated, the resultant oil layer was washed with water twice, to obtain 41.98 g of a mixed xylene solution of methyl trans-2,2-dimethyl-3-[(1E)-3-(hydroxyimino)-2-methyl-1-propenyl]cyclopropanecarboxylate as the hydroxyimino compound (3) (concentration: 49.60 wt%). Yield: 98.54% <Examples 4 to 8 and Reference Example 1>

A mixed xylene solution of methyl trans-2,2-dimethyl-3-[(1E)-3-(hydroxyimino)-2-methyl-1-propenyl]cyclopropanecarboxylate was obtained under the same conditions as in Example 3, excepting that conditions shown in Table 10 were changed and the dropping time of 23% sodium hydroxide was changed from 5.5 hours to 4.5 hours in Example 3. The results are shown in Table 11.

**Table 10**

| | Weight of mixed xylene solution of formyl compound (1) | Hydroxylamine hydrosulfate aqueous solution | Carboxylic acid (weight) | 23% Sodium hydroxide solution | Thermal retention time |
|---|---|---|---|---|---|
| Example 4 | 41.31 g | 37.64 g | myristic acid (2.28 g) | 21.47 g | 1.5 hours |
| Example 5 | 41.29 g | 37.68 g | hexanoic acid (1.18 g) | 21.03 g | 3.0 hours |
| Example 6 | 41.33 g | 37.66 g | pentanoic acid (1.12 g) | 22.69 g | 6.5 hours |
| Example 7 | 41.34 g | 37.6 g | butyric acid (0.94 g) | 22.72 g | 19.5 hours |
| Example 8 | 41.29 g | 37.67 g | propanoic acid (0.78 g) | 23.20 g | 31.5 hours |
| Reference Example 1 | 41.27 g | 37.71 g | not used | 21.29 g | 54.5 hours |

**Table 11**

| | Weight of mixed xylene solution of resultant compound (3) | Concentration of mixed xylene solution of resultant compound (3) | Yield |
|---|---|---|---|
| Example 4 | 44.54 g | 47.33 wt% | 99.79% |
| Example 5 | 41.49 g | 49.62 wt% | 97.52% |
| Example 6 | 39.99 g | 51.10 wt% | 96.69% |
| Example 7 | 40.36 g | 49.78 wt% | 95.11% |
| Example 8 | 39.52 g | 50.73 wt% | 94.95% |
| Reference Example 1 | 38.91 g | 50.38 wt% | 92.89% |

### <Example 9>

Under a nitrogen atmosphere, to 3.07 g of a solution prepared by dissolving methyl trans-2,2-dimethyl-3-[(1E)-3-(hydroxyimino)-2-methyl-1-propenyl]cyclopropanecarboxylate in mixed xylene (concentration of formyl compound (1): 32.74 wt%) were added 0.49 g of pyridine and 0.11 g of acetyl chloride. The resultant mixture was heated up to 100°C, to this was added 0.49 g of acetic anhydride and the mixture was thermally retained for 2 hours. The reaction mixture was cooled, then, diluted with mixed xylene, to obtain 9.60 g of a mixed xylene solution of methyl 2,2-dimethyl-3-[(1E)-2-cyano-1-propen-1-yl]cyclopropanecarboxylate (content of cyano compound (2): 9.11 wt%). Yield: 95.6%.

### <Example 10>

Under a nitrogen atmosphere, 42.35 g of mixed xylene, 20.65 g of pyridine, 3.19 g of acetyl chloride and 22.56 g of acetic anhydride were mixed. The resultant mixture was heated up to 100°C, and into this, 95.61 g of a solution prepared by dissolving methyl trans-2,2-dimethyl-3-[(1E)-3-(hydroxyimino)-2-methyl-1-propenyl]cyclopropanecarboxylate in mixed xylene (concentration of formyl compound (1): 44.20 wt%) was dropped over a period of 6 hours, then, the mixture was thermally retained for 2 hours. The reaction mixture was cooled, then, to this, 42.35 g of water and 12.15 g of 97 wt% sulfuric acid were added and mixed, and the mixture was liquid-separated to obtain an oil layer 1 and an aqueous layer 1. To the aqueous layer 1, 21.19 g of mixed xylene was added and mixed, the mixture was liquid-separated to obtain an oil layer 2 which was then combined with the oil layer 1 obtained previously. To this, 42.50 g of water and 21.98 g of 23 wt% sodium hydroxide were added and mixed, and the mixture was liquid-separated. The resultant oil layer 3 was further mixed with 42.31 g of water and the mixture was liquid-separated, and the resultant oil layer was condensed, to obtain 122.57 g of a mixed xylene solution of methyl 2,2-dimethyl-3-[(1E)-2-cyano-1-propen-1-yl]cyclopropanecarboxylate (content of cyano compound (2): 29.79 wt%).
Yield: 95.3%

### <Examples 11 to 14>

The same procedure as in Example 9 was carried out excepting that conditions shown in Table 12 were changed in Example 9, to obtain a mixed xylene solution of methyl 2,2-dimethyl-3-[(1E)-2-cyano-1-propen-1-yl]cyclopropanecarboxylate as the cyano compound (2). The results are shown in Table 13.

**Table 12**

| | Weight of compound (1) | Weight of mixed xylene | Weight of pyridine | Acid or acid generator (weight) | Weight of acetic anhydride |
|---|---|---|---|---|---|
| Example 11 | 1.00 g | 1.97 g | 0.47 g | 14 wt% hydrogen chloride dioxane solution (0.36 g) | 0. 63 g |
| Example 12 | 1.00 g | 2.00 g | 0.48 g | 30 wt% hydrogen bromide acetic acid solution (0.38 g) | 0.61 g |
| Example 13 | 1.01 g | 2.10 g | 0.49 g | aluminum chloride (0.03 g) | 0.64 g |
| Example 14 | 1.01 g | 1.97 g | 0.49 g | methanesulfoni c acid (0.14 g) | 0.63 g |

**Table 13**

| | Reaction time | Weight of xylene solution of compound (2) | Content of compound (2) in xylene solution of compound (2) | Yield of compound (2) |
|---|---|---|---|---|
| Example 11 | 2 hours | 3.76 g | 23.5 wt% | 98.4% |
| Example 12 | 2 hours | 6.83 g | 12.43 wt% | 94.2% |
| Example 13 | 1 hour | 10.04 g | 7.98 wt% | 87.6% |
| Example 14 | 6 hours | 6.97 g | 12.55 wt% | 97.1% |

### <Example 15>

Under a nitrogen atmosphere, 17.40 g of pyridine, 42.25 g of mixed xylene and 4.62 g of pyridinium chloride were mixed, and the resultant mixture was heated up to 100°C. Into this, 95.59 g of a solution prepared by dissolving methyl trans-2,2-dimethyl-3-[(lE)-3-(hydroxyimino)-2-methyl-1-propenyl]cyclopropanecarboxylate in mixed xylene (concentration of formyl compound (1): 44.20 wt%) was dropped and 26.54 g of acetic anhydride was dropped simultaneously, at the same temperature. Both the dropping time of the above-described solution and the dropping time of acetic anhydride were 6 hours. After completion of dropping, the resultant mixture was thermally retained for 2 hours. The mixture was cooled down to 20°C, then, to this, 42.25 g of water and 12.13 g of 97 wt% sulfuric acid were added and mixed, and the mixture was liquid-separated to obtain an oil layer 1 and an aqueous layer 1. To the aqueous layer 1, 21.13 g of mixed xylene was added and mixed, the mixture was liquid-separated to obtain an oil layer 2 which was then combined with the oil layer 1 obtained previously. To this, 42.25 g of water and 24.35 g of 23 wt% sodium hydroxide were added and mixed, and the mixture was liquid-separated. The resultant oil layer 3 was further mixed with 42.25 g of water and the mixture was liquid-separated, and the resultant oil layer was condensed, to obtain 82.26 g of a solution containing methyl 2,2-dimethyl-3-[(1E)-2-cyano-1-propen-1-yl]cyclopropanecarboxylate (content of cyano compound (2): 44.69 wt%). Yield: 95.0%

### <Example 16>

Under a nitrogen atmosphere, 17.40 g of pyridine, 42.25 g of mixed xylene and 4.62 g of pyridinium chloride were mixed, the resultant mixture was heated up to 100°C, then, 1.06 g of acetic anhydride was added. Into this, 87.97 g of a solution prepared by dissolving methyl trans-2,2-dimethyl-3-[(1E)-3-(hydroxyimino)-2-methyl-1-propenyl]cyclopropanecarboxylate in mixed xylene (concentration of formyl compound (1): 47.80 wt%) was dropped and 25.52 g of acetic anhydride was dropped simultaneously at the same temperature. Both the dropping time of the above-described solution and the dropping time of acetic anhydride were 6 hours. After completion of dropping, the resultant mixture was thermally retained for 2 hours. The mixture was cooled down to 20°C, then, to this, 42.25 g of water and 11.10 g of 97 wt% sulfuric acid were added and mixed, and the mixture was liquid-separated to obtain an oil layer 1. To this, 42.25 g of water and 24.35 g of 23 wt% sodium hydroxide were added and mixed, and the mixture was liquid-separated. The resultant oil layer 2 was further mixed with 42.25 g of water and the mixture was liquid-separated, and the resultant oil layer was condensed, to obtain 64.51 g of a solution containing methyl 2,2-dimethyl-3-[(1E)-2-cyano-1-propen-1-yl]cyclopropanecarboxylate (content of cyano compound (2): 56.09 wt%). Yield94.1%

### <Example 17>

Under a nitrogen atmosphere, 3.96 g of pyridine, 52.82 g of mixed xylene and 5.19 g of 35% hydrochloric acid were mixed, then, pressure was reduced to 5 kPa, and dehydrated under reflux with heating, to obtain a xylene solution containing pyridinium chloride. To the resultant xylene solution was added 21.75 g of pyridine, and the mixture was heated up to 100°C. Into this, 109.62 g of a solution prepared by dissolving methyl trans-2,2-dimethyl-3-[(1E)-3-(hydroxyimino)-2-methyl-1-propenyl]cyclopropanecarboxylate in mixed xylene (concentration of formyl compound (1): 48.18 wt%) was dropped and 30.63 g of acetic anhydride was dropped simultaneously at the same temperature. Both the dropping time of the above-described solution and the dropping time of acetic anhydride were 6 hours. After completion of dropping, the resultant mixture was thermally retained for 2 hours, to obtain 205.44 g of a solution containing methyl 2,2-dimethyl-3-[(1E)-2-cyano-1-propen-1-yl]cyclopropanecarboxylate (content of compound (2): 22.15 wt%). Yield: 94.2%

### <Reference Example 1>

Under a nitrogen atmosphere, 1.00 g of methyl trans-2,2-dimethyl-3-[(1E)-3-(hydroxyimino)-2-methyl-1-propenyl]cyclopropanecarboxylate (purity: 98.55 wt%) was dissolved in 2.48 g of mixed xylene, and to the resultant solution was added 0.49 g of pyridine. The resultant mixture was heated up to 100°C, to this was added 0.64 g of acetic anhydride and the mixture was thermally retained for 16 hours. Methyl trans-2,2-dimethyl-3-[(1E)-3-(acetoxyimino)-2-methyl-1-propenyl]cyclopropanecarboxylate as a reaction intermediate remained in a proportion of 14% as the area percentage with respect to the sum with methyl 2,2-dimethyl-3-[(1E)-2-cyano-1-propen-1-yl]cyclopropanecarboxylate. After cooling, it was diluted with mixed xylene, to obtain 8.83 g of a mixed xylene solution of methyl trans-2,2-dimethyl-3-[(1E)-3-(acetoxyimino)-2-methyl-1-propenyl]cyclopropanecarboxylate and methyl 2,2-dimethyl-3-[(1E)-2-cyano-1-propen-1-yl]cyclopropanecarboxylate (content: 8.31 wt%). Yield: 80.3%.

### Industrial Applicability

Cyclopropanecarboxylic acid ester compounds such as hydroxyiminoalkenylcyclopropanecarboxylic acid esters, cyanoalkenylcyclopropane compounds and the like are compounds important as a synthetic intermediate for compounds for controlling pests such as harmful insects and the like.

The present invention can be used as a production method of such compounds.

## Claims

1. A method comprising
an oximation step of reacting a formylalkenylcyclopropanecarboxylic acid ester represented by the formula (1): 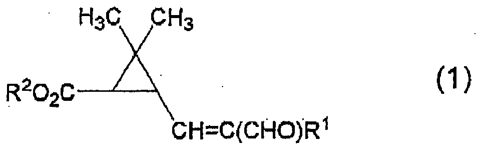 (wherein, R¹ represents an alkyl group optionally having substituent(s) or halogen atom(s), and R² represents an alkyl group optionally having substituent(s) or a benzyl group optionally having substituent(s))
and hydroxylamine in a solvent in the presence at least one compound selected from the group consisting of carboxylic acids and carboxylic acid metal salts,
to produce a hydroxyiminoalkenylcyclopropanecarboxylic acid ester represented by the formula (3): 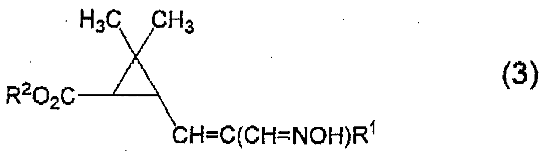 (wherein, R¹ and R² are as defined above).

2. The production method according to Claim 1, wherein the at least one compound selected from the group consisting of carboxylic acids and carboxylic acid metal salts is a carboxylic acid having 3 or more carbon atoms or a metal salt of a carboxylic acid having 3 or more carbon atoms.

3. The production method according to Claim 1 or 2, wherein the solvent is a mixed solvent composed of water and an organic solvent immiscible with water.

4. A method comprising
an oximation step of reacting a formylalkenylcyclopropanecarboxylic acid ester represented by the formula (1): 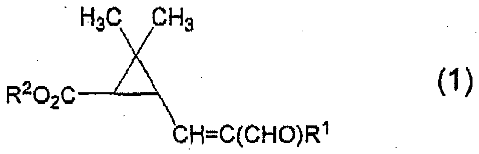 (wherein, R¹ represents an alkyl group optionally having substituent(s) or halogen atom(s), and R² represents an alkyl group optionally having substituent(s) or a benzyl group optionally having substituent(s))
and hydroxylamine in a solvent in the presence of at least one compound selected from the group consisting of carboxylic acids and carboxylic acid metal salts,
and a nitrilation step of reacting a hydroxyiminoalkenylcyclopropanecarboxylic acid ester represented by the formula (3): 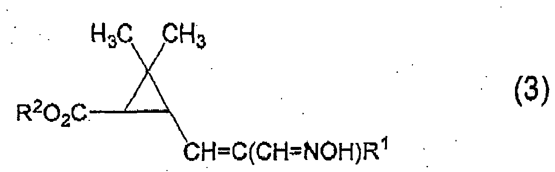 (wherein, R¹ and R² are as defined above) obtained in said oximation step and a dehydrating agent,
to produce a cyanoalkenylcyclopropanecarboxylic acid ester represented by the formula (2): 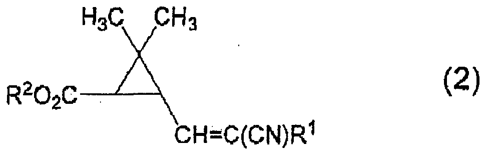 (wherein, R¹ and R² are as defined above).

5. The production method according to Claim 4, wherein the dehydrating agent is at least one compound selected from the group consisting of carboxylic anhydrides and acyl halides, preferably a carboxylic anhydride.

6. The production method according to Claim 4 or 5, wherein the nitrilation step is carried out in the presence of at least one acid selected from the group consisting of hydrogen halides and organic sulfonic acids.

7. The production method according to Claim 4 or 5, wherein the nitrilation step is carried out in the presence of an organic base.

8. The production method according to Claim 4, further comprising a step of removing at least one compound selected from the group consisting of carboxylic acids and carboxylic acid metal salts from a mixture obtained by the reaction in the nitrilation step.

9. The production method according to Claim 8, wherein the at least one compound selected from the group consisting of carboxylic acids and carboxylic acid metal salts is removed into an alkali aqueous solution, preferably having pH of 10 or more.

10. The production method according to Claim 9, wherein the alkali aqueous solution is an alkali metal hydroxide aqueous solution or an alkali metal carbonate aqueous solution.

11. A method comprising
a step of reacting a hydroxyiminoalkenylcyclopropane compound represented by the formula (3): 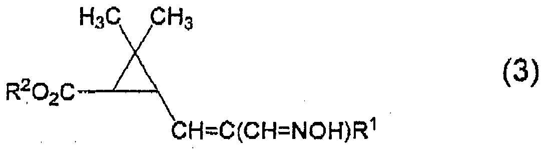 (wherein, R¹ represents an alkyl group optionally having substituent(s) or halogen atom(s), and R² represents an alkyl group optionally having substituent(s) or a benzyl group optionally having substituent(s))
and a carboxylic anhydride in the presence of an organic base and at least one acid selected from the group consisting of hydrogen halides and organic sulfonic acids,
to produce a cyanoalkenylcyclopropane compound represented by the formula (2): 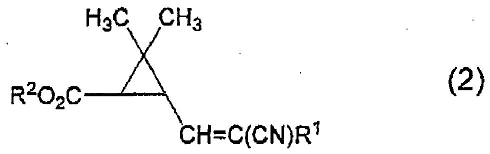 (wherein, R¹ and R² are as defined above).

12. The production method according to Claim 11, wherein said step is a step of reacting a hydroxyiminoalkenylcyclopropane compound represented by the formula (1) and a carboxylic anhydride in the presence of a solvent.

13. The production method according to Claim 12, wherein the organic base forms a salt of the acid with the organic base.

14. The production method according to Claim 11 or 12, wherein the carboxylic anhydride is a carboxylic anhydride having 2 to 8 carbon atoms, preferably acetic anhydride.

15. The production method according to Claim 11 or 12, wherein the organic base is at least one base selected from the group consisting of pyridine, methylethylpyridine, dimethylaminopyridine and triethylamine, preferably pyridine.

16. The production method according to Claim 11 or 12, wherein said acid is a hydrogen halide, preferably hydrogen chloride.

17. The production method according to Claim 11 or 12, wherein said acid is an acid generated from at least one compound selected from the group consisting of carboxylic halides, organic sulfonic halides, metal halides and thionyl halides.

## Patentansprüche

1. Ein Verfahren, umfassend
einen Oximierungsschritt des Umsetzens eines Formylalkenylcyclopropailcarbonsäureesters, dargestellt durch die Formel (1): 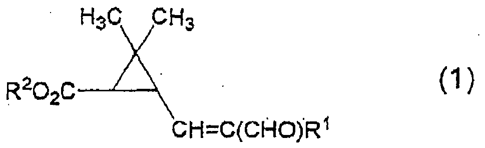 (wobei R¹ einen Alkylrest, der gegebenenfalls (einen) Substituenten aufweist, oder (ein) Halogenatom(e) darstellt, und R² einen Alkylrest, der gegebenenfalls (einen) Substituenten aufweist, oder einen Benzylrest, der gegebenenfalls (einen) Substituenten aufweist, darstellt)
und Hydroxylamin in einem Lösungsmittel in Gegenwart mindestens einer Verbindung, ausgewählt aus der Gruppe bestehend aus Carbonsäuren und Carbonsäuremetallsalzen, um einen Hydroxyiminoalkenylcyclopropancarbonsäureester, dargestellt durch die Formel (3), herzustellen: 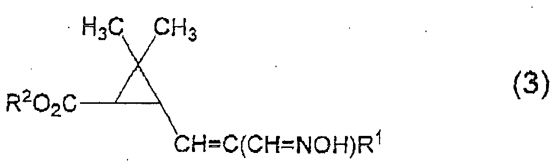 (wobei R¹ und R² wie vorstehend definiert sind).

2. Das Herstellungsverfahren nach Anspruch 1, wobei die mindestens eine Verbindung, ausgewählt aus der Gruppe bestehend aus Carbonsäuren und Carbonsäuremetallsalzen, eine Carbonsäure mit 3 oder mehr Kohlenstoffatomen oder ein Metallsalz einer Carbonsäure mit 3 oder mehr Kohlenstoffatomen ist.

3. Das Herstellungsverfahren nach Anspruch 1 oder 2, wobei das Lösungsmittel ein Mischlösungsmittel ist, zusammengesetzt aus Wasser und einem in Wasser unlöslichen organischen Lösungsmittel.

4. Ein Verfahren, umfassend
einen Oximierungsschritt des Umsetzens eines Formylalkenylcyclopropancarbonsäureesters, dargestellt durch die Formel (1): 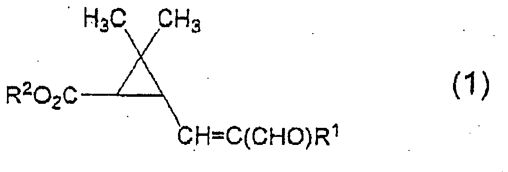 (wobei R¹ einen Alkylrest, der gegebenenfalls (einen) Substituenten aufweist, oder (ein) Halogenatom(e) darstellt, und R² einen Alkylrest, der gegebenenfalls (einen) Substituenten aufweist, oder einen Benzylrest, der gegebenenfalls (einen) Substituenten aufweist, darstellt)
und Hydroxylamin in einem Lösungsmittel in Gegenwart mindestens einer Verbindung, ausgewählt aus der Gruppe bestehend aus Carbonsäuren und Carbonsäuremetallsalzen, und einen Nitrilierungsschritt des Umsetzens eines Hydroxyiminoalkenylcyclopropancarbonsäureesters, dargestellt durch die Formel (3): 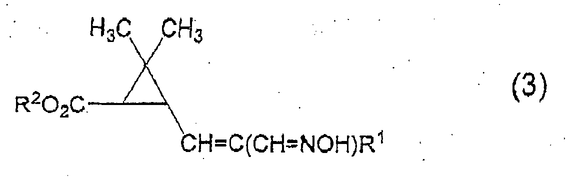 (wobei R¹ und R² wie vorstehend definiert sind), der in dem Oximierungsschritt erhalten wurde, und eines Dehydrierungsmittels,
um ein Cyanoalkenylcyclopropancarbonsäureester, dargestellt durch die Formel (2), herzustellen: 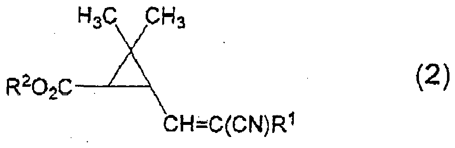 (wobei R¹ und R² wie vorstehend definiert sind).

5. Das Herstellungsverfahren nach Anspruch 4, wobei das Dehydrierungsmittel mindestens eine Verbindung ist, ausgewählt aus der Gruppe bestehend aus Carbonsäureanhydriden und Acylhalogeniden, vorzugsweise einem Carbonsäureanhydrid.

6. Das Herstellungsverfahren nach Anspruch 4 oder 5, wobei der Nitrilierungsschritt in Gegenwart mindestens einer Säure, ausgewählt aus der Gruppe bestehend aus Halogenwasserstoffen und organischen Sulfonsäuren, durchgeführt wird.

7. Das Herstellungsverfahren nach Anspruch 4 oder 5, wobei der Nitrilierungsschritt in Gegenwart einer organischen Base durchgeführt wird.

8. Das Herstellungsverfahren nach Anspruch 4, außerdem umfassend einen Schritt des Entfernens mindestens einer Verbindung, ausgewählt aus der Gruppe bestehend aus Carbonsäuren und Carbonsäuremetallsalzen, aus einem Gemisch, der durch die Umsetzung im Nitrilierungsschritt erhalten wurde.

9. Das Herstellungsverfahren nach Anspruch 8, wobei die mindestens eine Verbindung, ausgewählt aus der Gruppe bestehend aus Carbonsäuren und Carbonsäuremetallsalzen, in eine wässrige Alkalilösung entfernt wird, die vorzugsweise einen pH von 10 oder mehr aufweist.

10. Das Herstellungsverfahren nach Anspruch 9, wobei die wässrige Alkalilösung eine wässrige Alkalimetallhydroxidlösung oder eine wässrige Alkalimetallcarbonatlösung ist.

11. Ein Verfahren, umfassend
einen Schritt des Umsetzens einer Hydroxyiminoalkenylcyclopropanverbindung, dargestellt durch die Formel (3): 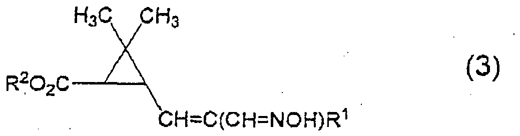 (wobei R¹ einen Alkylrest, der gegebenenfalls (einen) Substituenten aufweist, oder (ein) Halogenatom(e) darstellt, und R² einen Alkylrest, der gegebenenfalls (einen) Substituenten aufweist, oder einen Benzylrest, der gegebenenfalls (einen) Substituenten aufweist, darstellt)
und eines Carbonsäureanhydrids in Gegenwart einer organischen Base und mindestens einer Säure, ausgewählt aus der Gruppe bestehend aus Halogenwasserstoffen und organischen Sulfonsäuren,
um eine Cyanoalkenylcyclopropanverbindung, dargestellt durch die Formel (2), herzustellen: 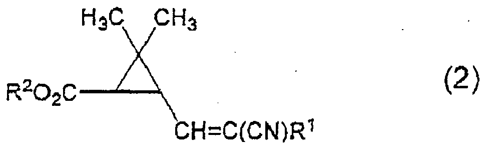 (wobei R¹ und R² wie vorstehend definiert sind).

12. Das Herstellungsverfahren nach Anspruch 11, wobei der Schritt ein Schritt des Umsetzens einer Hydroxyiminoalkenylcyclopropanverbindung, dargestellt durch die Formel (1), und eines Carbonsäureanhydrids in Gegenwart eines Lösungsmittels, ist.

13. Das Herstellungsverfahren nach Anspruch 12, wobei die organische Base ein Salz der Säure mit der organischen Base bildet.

14. Das Herstellungsverfahren nach Anspruch 11 oder 12, wobei das Carbonsäureanhydrid ein Carbonsäureanhydrid mit 2 bis 8 Kohlenstoffatomen, vorzugsweise Essigsäureanhydrid, ist.

15. Das Herstellungsverfahren nach Anspruch 11 oder 12, wobei die organische Base mindestens eine Base, ausgewählt aus der Gruppe bestehend aus Pyridin, Methylethylpyridin, Dimethylaminopyridin und Triethylamin, vorzugsweise Pyridin, ist.

16. Das Herstellungsverfahren nach Anspruch 11 oder 12, wobei die Säure ein Halogenwasserstoff, vorzugsweise Chlorwasserstoff, ist.

17. Das Herstellungsverfahren nach Anspruch 11 oder 12, wobei die Säure eine Säure ist, die aus mindestens einer Verbindung, ausgewählt aus der Gruppe bestehend aus Carbonsäurehalogeniden, organischen Sulfonsäurehalogeniden, Metallhalogeniden und Thionylhalogeniden erzeugt wurde.

## Revendications

1. Méthode comprenant :
une étape d'oximation faisant réagir un ester d'acide formylalcénylcyclopropanecarboxylique représenté par la formule (1) : 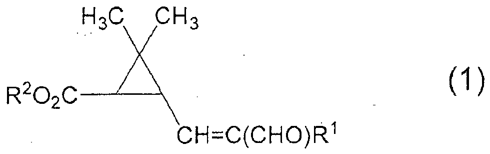 (dans laquelle, R¹ représente un groupe alkyle comportant éventuellement un ou plusieurs substituants ou atomes d'halogène, et R² représente un groupe alkyle comportant éventuellement un ou plusieurs substituants ou un groupe benzyle comportant éventuellement un ou plusieurs substituants)
et de l'hydroxylamine dans un solvant en présence d'au moins un composé choisi dans le groupe constitué des acides carboxyliques et des sels métalliques d'acides carboxyliques,
pour produire un ester d'acide hydroxyiminoalcénylcyclopropanecarboxylique représenté par la formule (3) : 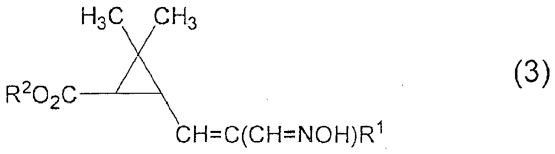 (dans laquelle, R¹ et R² sont tels que définis ci-dessus).

2. Méthode de production selon la revendication 1, dans laquelle ledit au moins un composé choisi dans le groupe constitué des acides carboxyliques et des sels métalliques d'acides carboxyliques est un acide carboxylique comportant 3 atomes de carbone ou plus ou un sel métallique d'un acide carboxylique comportant 3 atomes de carbone ou plus.

3. Méthode de production selon la revendication 1 ou 2, dans laquelle le solvant est un solvant mixte composé d'eau et d'un solvant organique non miscible à l'eau.

4. Méthode comprenant
une étape d'oximation faisant réagir un ester d'acide formylalcénylcyclopropanecarboxylique représenté par la formule (1) : 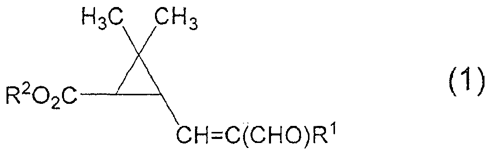 (dans laquelle, R¹ représente un groupe alkyle comportant éventuellement un ou plusieurs substituants ou un ou plusieurs atomes d'halogène, et R² représente un groupe alkyle comportant éventuellement un ou plusieurs substituants ou un groupe benzyle comportant éventuellement un ou plusieurs substituants)
et de l'hydroxylamine dans un solvant en présence d'au moins un composé choisi dans le groupe constitué d'acides carboxyliques et de sels métalliques d'acides carboxyliques,
et une étape de nitrilation faisant réagir un ester d'acide hydroxyiminoalcénylcyclopropanecarboxylique représenté par la formule (3) : 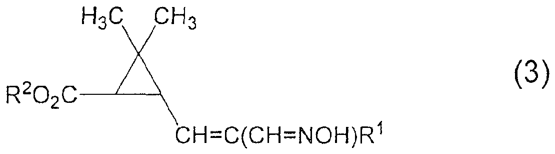 (dans laquelle, R¹ et R² sont tels que définis ci-dessus) obtenu par ladite étape d'oximation et un agent déshydratant,
pour produire un ester d'acide cyanoalcénylcyclopropanecarboxylique représenté par la formule (2) : 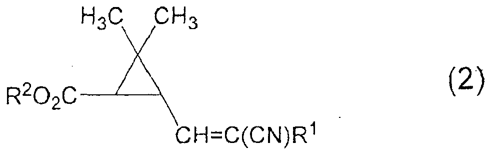 (dans laquelle, R¹ et R² sont tels que définis ci-dessus).

5. Méthode de production selon la revendication 4, dans laquelle l'agent déshydratant est au moins un composé choisi dans le groupe constitué des anhydrides carboxyliques et des halogénures d'acyle, de préférence un anhydride carboxylique.

6. Méthode de production selon la revendication 4 ou 5, dans laquelle l'étape de nitrilation est réalisée en présence d'au moins un acide choisi dans le groupe constitué des halogénures d'hydrogène et des acides sulfoniques organiques.

7. Méthode de production selon la revendication 4 ou 5, dans laquelle l'étape de nitrilation est réalisée en présence d'une base organique.

8. Méthode de production selon la revendication 4, comprenant en outre une étape d'élimination d'au moins un composé choisi dans le groupe constitué des acides carboxyliques et des sels métalliques d'acides carboxyliques à partir d'un mélange obtenu par la réaction de l'étape de nitrilation.

9. Méthode de production selon la revendication 8, dans laquelle ledit au moins un composé choisi dans le groupe constitué des acides carboxyliques et des sels métalliques d'acides carboxyliques est éliminé dans une solution aqueuse alcaline, ayant de préférence un pH de 10 ou plus.

10. Méthode de production selon la revendication 9, dans laquelle la solution aqueuse alcaline est une solution aqueuse d'hydroxyde de métal alcalin ou une solution aqueuse de carbonate de métal alcalin.

11. Méthode comprenant
une étape faisant réagir un composé d'hydroxyiminoalcénylcyclopropane représenté par la formule (3) : 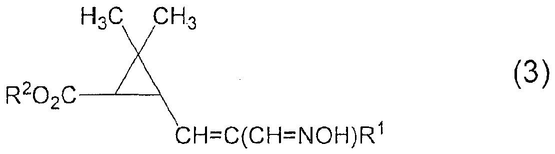 (dans laquelle, R¹ représente un groupe alkyle comportant éventuellement un ou plusieurs substituants ou un ou plusieurs atomes d'halogène, et R² représente un groupe alkyle comportant éventuellement un ou plusieurs substituants ou un groupe benzyle comportant éventuellement un ou plusieurs substituants)
et un anhydride carboxylique en présence d'une base organique et d'au moins un acide choisi dans le groupe constitué des halogénures d'hydrogène et des acides sulfoniques organiques,
pour produire un composé de cyanoalcénylcyclopropane représenté par la formule (2) : 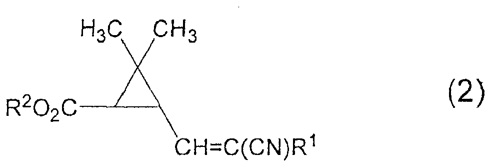 (dans laquelle, R¹ et R² sont tels que définis ci-dessus).

12. Méthode de production selon la revendication 11, dans laquelle ladite étape est une étape faisant réagir un composé d'hydroxyiminoalcénylcyclopropane représenté par la formule (1) et un anhydride carboxylique en présence d'un solvant.

13. Méthode de production selon la revendication 12, dans laquelle la base organique forme un sel de l'acide avec la base organique.

14. Méthode de production selon la revendication 11 ou 12, dans laquelle l'anhydride carboxylique est un anhydride carboxylique comportant 2 à 8 atomes de carbone, de préférence l'anhydride acétique.

15. Méthode de production selon la revendication 11 ou 12, dans laquelle la base organique est au moins une base choisie dans le groupe constitué de la pyridine, de la méthyléthylpyridine, du diméthylaminopyridine et du triéthylamine, de préférence la pyridine.

16. Méthode de production selon la revendication 11 ou 12, dans laquelle ledit acide est un halogénure d'hydrogène, de préférence le chlorure d'hydrogène.

17. Méthode de production selon la revendication 11 ou 12, dans laquelle ledit acide est un acide généré à partir d'au moins un composé choisi dans le groupe constitué des halogénures carboxyliques, des halogénures sulfoniques organiques, des halogénures métalliques et des halogénures de thionyle.
